(19) Europäisches Patentamt
European Patent Office
Office européen des brevets

(11) EP 0 778 944 B1

(12) **EUROPEAN PATENT SPECIFICATION**

(45) Date of publication and mention of the grant of the patent:
**03.11.1999 Bulletin 1999/44**

(21) Application number: **95931685.2**

(22) Date of filing: **31.08.1995**

(51) Int Cl.6: **G01N 33/49**, B01L 3/14

(86) International application number:
**PCT/US95/11169**

(87) International publication number:
**WO 96/07097 (07.03.1996 Gazette 1996/11)**

(54) **CELL SEPARATION APPARATUS AND METHOD**

VORRICHTUNG UND VERFAHREN ZUR TRENNUNG VON ZELLEN

DISPOSITIF ET PROCEDE DE SEPARATION DE CELLULES

(84) Designated Contracting States:
**AT BE CH DE DK ES FR GB GR IE IT LI LU MC NL PT SE**
Designated Extension States:
**LT LV SI**

(30) Priority: **31.08.1994 US 299465**
**31.08.1994 US 299468**
**31.08.1994 US 299469**

(43) Date of publication of application:
**18.06.1997 Bulletin 1997/25**

(73) Proprietor: **DENDREON CORPORATION**
**Seattle, WA 98121 (US)**

(72) Inventor: **VAN VLASSELAER, Peter**
**Sunnyvale, CA 94087 (US)**

(74) Representative: **VOSSIUS & PARTNER**
**Siebertstrasse 4**
**81675 München (DE)**

(56) References cited:

**EP-A- 0 198 462**
**DE-A- 2 115 032**
**US-A- 5 314 074**
**WO-A-94/17209**
**US-A- 4 927 750**

- **EXPERIMENTAL HEMATOLOGY, vol. 23, 1995 pages 1024-1029, SCHRIBER ET AL. 'Enrichment of bone marrow and blood progenitor (CD34+) cells by density gradients with sufficient yields for transplantation'**
- **SCANDINAVIAN JOURNAL OF HAEMATOLOGY, vol. 24, 1980 COPENHAGEN, DK, pages 254-262, OLOFSSON ET AL. 'Separation of human bone marrow cells in density gradients of polyvinylpyrrolidone coated silica gel (Percoll)' cited in the application**
- **EXPERIMENTAL HEMATOLOGY, vol. 13, no. 7, August 1985 pages 680-684, LASKY ET AL. 'Size and density characterization of human committed and multipotent hematopoietic progenitors' cited in the application**
- **JOURNAL OF REPRODUCTIVE MEDICINE, vol. 37, no. 5, May 1992 pages 410-418, HOLZGREVE ET AL. 'Fetal cells in the maternal circulation' cited in the application**
- **EXPERIMENTAL HEMATOLOGY, vol. 16, 1988 pages 190-194, KIES ET AL. 'Autologous bone marrow transplantation in breast cancer: separation of clonogenic tumor cell colonies by gradient fractionation'**
- **PROCEEDINGS OF THE NATIONAL ACADEMY OF SCIENCES OF USA, vol. 84, no. 20, October 1987 WASHINGTON US, pages 7295-7299, RESNICOFF ET AL. 'Subpopulations of MCF7 cells separated by Percoll gradient centrifugation: a model to analyze the heterogeneity of human breast cancer'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 778 944 B1

**Description**

1. FIELD OF THE INVENTION

The present invention relates to methods of enriching for a desired cell population from cell sources, such as body fluids, dispersed tissue specimens and cultured cells. In particular, the method uses a cell-trap centrifugation apparatus which contains a defined density medium for separating lower density cells from cell populations. The method may be augmented by selectively increasing the densities of unwanted low density cells using microparticles having covalently attached cell attachment molecules.

2. BACKGROUND

Isolating specific cell types from biological fluids and tissues is often desirable for clinical diagnostic and therapeutic applications. In the clinical diagnostics field, there is a need, for example, for morphological analysis of tumor cells, fetal karyoty-ping, and tissue typing procedures. Therapeutically, there is a need, for example, for purging cells or tissues intended for use in autologous cellular or tissue transfusions or transplantations, *e.g.* purging tissues of viral antigens and tumor cells. There is also a need for enriching or isolating desirable cells for use in transplantations, *e.g.* for use in *ex vivo* expansion of hematopoietic cells intended for allogeneic and autologous transplantation.

Several methods are known in the art for separating desirable cells from body fluids. Such methods include separating cells based upon buoyant density in a cell separation composition (U.S. Pat. No. 4,927,750), separating serological factors on density gradients using latex beads coated with anti-serological factor (U.S. Pat. No. 3,862,303), separating cells through the use of a magnetic field (U.S. Pat. No. 4,777,145), and separating T and B cells on density gradients (U.S. Pat. No. 4,511,662). Cell separation methods known in the art may have the disadvantage of cell loss due to the sticking of cells to tubes and pipettes.

There is need for rapid and efficient means of isolating relatively rare populations of cells for diagnostic and therapeutic procedures. The present invention fills this need by providing methods of isolating or enriching minor populations of desirable cells from cell sources or mixtures containing multiple cell populations. Moreover, the invention provides for collection of enriched cells in a high yield.

Specifically, it is the discovery of the present invention that by providing highly defined cell separation media having precisely measured specific densities, specific, defined populations of cells can be isolated. Moreover, the invention provides a cell-trap centrifugation apparatus that greatly enhances collection of cells by this process. Alternatively or in addition, the process is enhanced by use of a density adjusted cell sorting step (DACS), to provide a higher level of specificity to the separation process. The invention also provides specific methods for isolation of three specific cell types that are important in diagnostic and therapeutic methods -- fetal nucleated cells, hematopoietic progenitor ($CD34^+$) cells, and breast tumor cells.

3. SUMMARY OF THE INVENTION

In one aspect, the invention is directed to a cell-separation apparatus useful in isolation of cells by density methods. The apparatus includes a centrifugation tube that has a constricted region situated within the tube. The constriction member is constructed and positioned to retain fluid in the bottom portion of the tube when the tube is inverted. This feature permits decantation of the tube without substantial mixing of contents between compartments. The constriction preferably defines one or more downwardly sloped surfaces having lower edge regions defining an opening, which may be any shape, or which may form a plurality of openings, so long as it operates to retain fluid upon tube inversion. In a preferred embodiment, the constriction is an annular ring. Preferably, the annulus is constructed for forced fit into the tube for variable positioning within the tube.

The apparatus also includes a cell separation medium contained in the bottom portion of the tube. The medium is present in the tube to a level above the opening formed by the constriction. In this way, cells that are captured at an interface between the cell-separation medium and a lower density cell-loading medium can be discharged with the lower-density medium when the tube is inverted, without mixing with the contents of the bottom portion of the tube.

In a more specific embodiment, the centrifuge apparatus includes a tube with an annular member disposed therein. The annular member defines an opening having an area less than the area of a cross section of the tube. The annular member may be integrally formed in the tube, or it may be movable along the internal length of the tube, for adjustment of volumes. The tube also contains a density gradient solution which fills the lower portion and a part of the upper portion of the tube to a level at least above the opening in the annular member. For isolation of a specific cell type, the density gradient solution has an osmolality of $280 \pm 10$ m0sm/kg $H_2O$ and a specific density within 0.0005 gr/ml of the specific density of the desired cells.

Specific embodiments of the apparatus of the invention include a closed system, which includes a tube having

a constriction member and a closed top. Ports in the top serve as conduits for introduction of fluid into this tube embodiment, which may also include a port that communicates to the bottom portion of the tube. Alternatively, another embodiment of the apparatus of the invention is a centrifugable syringe described herein. The syringe includes a plunger with a constricted region that forms a fluid receiving space in the syringe bottom similar to the fluid receiving space formed by the constriction in the above-described tube apparatus.

The invention also includes the above-described apparatus in specific configurations defined for isolation or enrichment of specific cell types. For isolation of specific cells, the apparatus includes cell separation medium having a specific density that is within at least ±0.0005 gr/ml, and preferably within at least ±0.0002 gr/ml of the specific density of the desired cells. In preferred embodiments, for isolation of $CD34^+$ hematopoietic progenitor cells the medium will have an osmolality of 280±10 m0sm/kg $H_2O$ and a specific density of 1.0605 gr/ml; for isolation of fetal nucleated cells from maternal blood the specific density has an osmolality of 280±10 m0sm/kg $H_2O$ and a specific density of 1.0720 gr/ml; and for isolation of breast tumor cells, the separation medium has an osmolality of 280±10 m0sm/kg $H_2O$ and a specific density selected from the range 1.0490-1.0580 gr/ml, and more preferably, 1.0580 gr/ml.

In a related embodiment, the invention includes methods of isolating selected cells from cell mixtures, according using the above-defined apparatus. The method includes adding a cell mixture to the apparatus, centrifuging the apparatus at a gravitational force sufficient to pellet cells having specific densities greater than the specific density of the density gradient material in said tube, and collecting selected cells from the upper portion of the tube.

The method of the invention further includes, in specific embodiments, methods of isolating or enriching $CD34^+$ hematopoietic progenitor cells, breast tumor cells, and fetal nucleated cells from cell mixtures. Based on the applicant's discovery that highly defined media can be used to isolate specific cell types, the invention includes using the specific density and osmolality conditions described above, i.e., using media defined to ±0.0005, or preferably to ±0.0002 gr/ml, to isolate cells in a single specific density medium. $CD34^+$ cells that can be isolated include colony forming cells and cells with long term initiating capacity. Fetal nucleated cells include nucleated red blood cells and trophoblasts. In another embodiment, the invention includes a method of isolating natural killer cells or natural suppressor cells using a medium having a density of 1.0605±0.0005 gr/ml and an osmolarity of 280±10 m0sm/kg $H_2O$.

In yet another embodiment, the method of the invention includes a incubating the cell mixture from which cells are to be isolated with cell type-specific binding agents linked to carrier particles prior to centrifugation through cell separation medium. In a preferred embodiment, the particles will have a specific density that is at least 0.001 gr/ml greater than the specific density of said cell separation medium, for sedimentation of unwanted cells to which the binding agents are directed. Binding agents used in this aspect of the invention may include antibodies, lectins, cytokines and the like. A specific binding agent that is useful in depleting leukocytes is anti-CD45 antibody. Alternatively, the specific binding agents can be directed to the cells of interest for selective sedimentation thereof. Specific embodiments include carrier particles formed of silane-activated silica, and preferably 3-aminopropyltriethoxy silane-activated silica.

## 4. BRIEF DESCRIPTION OF THE DRAWINGS

FIGS. 1A-C show cross-sectional views of a centrifugation apparatus of the invention, illustrating the steps of isolating or separating cells according to one of the methods of the invention;

FIGS. 2A and 2B show schematic cross-sectional (2A) and perspective (2B) views of an embodiment of the centrifuge tube apparatus that includes a shield;

FIG. 3 shows a cross-sectional view of an alternative embodiment of the constriction member of the centrifuge apparatus with a valve;

FIGS. 4A-F show cross-sectional views of alternative embodiments of the lower portion of the tube and constriction member of the invention;

FIGS. 5A and 5B show cross-sectional views of further alternative embodiments of the invention having multiple constriction members;

FIG. 6 shows an alternative embodiment of the centrifuge tube apparatus in a closed system suitable for processing of sterile specimens;

FIG. 7 shows a centrifugable syringe embodiment of the centrifuge apparatus of the invention;

FIGS. 8(A-D) show a schematic drawings comparing conventional (8 A,B) with density adjusted cell sorting procedure (8 C,D);

FIGS. 9A-9C shows a comparison of cell numbers in three cell preparations isolated by the conventional method using "FICOLL" as the density material (Fig. 9A), "FICOLL" plus cell-trap tubes (Fig. 9B), and adjusted "PERCOLL" density gradient plus cell-trap tubes (Fig. 9C);

FIG. 10 shows the distribution of colony forming units (CFU's) in interface and pellet fractions;

FIG. 11 shows distribution of different types of CFU's in interface and pellet fractions;
FIG. 12 shows distribution of long-term culture initiating capability (LTC-IC) in interface and pellet fractions;
FIG. 13 shows distribution of T cells in interface and pellet fractions;
FIG. 14 shows distribution of natural suppressor activity in different density fractions;
FIG. 15 shows distribution of natural killer activity in different density fractions;
FIGS. 16A-16F show flow cytometric analysis of $CD34^+$ cell enrichment after density gradient centrifugation plus density adjusted cell sorting;
FIGS. 17A-17D illustrate the enrichment of 4 types of breast tumor cells using the cell separation method of the present invention; and
FIGS. 18A-18D illustrate breast tumor cell enrichment of breast tumor cells spiked in a cell at a specific density of 1.0580 g/ml.

Trademarked terms are identified herein by upper case letters and enclosure in quotation marks.

## 5. DETAILED DESCRIPTION OF THE INVENTION

The present invention relates to methods of rapid and high yield isolation or enrichment of a desired cell population from body fluids, dispersed tissue specimens, cultured cells and their components. The method is based on density gradient centrifugation of selected cell mixtures using highly defined density gradient media. More specifically, the present invention includes a specially designed cell-trap centrifugation tube apparatus containing density gradient solution that maximizes yield and improves efficiency of the collection process.

The general invention also includes, and is exemplified by, cell separation methods for isolation of specific cell types: fetal cells, hematopoietic progenitor $CD34^+$ cells, and breast tumor cells. Each of these cell types illustrates an important diagnostic and/or therapeutic use of cells isolated by the methods of the invention. For example, as described herein, nucleated fetal cells can be isolated from circulating maternal blood for the purpose of performing a variety of genetic analyses, *e.g.* karyotyping. Breast tumor cells can be isolated from circulating blood for the purpose of performing diagnostic tests, *e.g.* cytological examination, or for the purpose of purging tumor cells from a blood sample intended for subsequent re-infusion, *e.g.* transfusions or transplants. Hematopoietic progenitor cells can be isolated from blood or bone marrow for use as donor cells in bone marrow transplantation.

### 5.1 DENSITY GRADIENT CELL SEPARATION METHOD

This section describes cell separation methods using specific density gradients in accordance with the invention. More specifically, the sections that follow contain descriptions of particular aspects of the invention: (1) the use of a cell-trap centrifugation tube in conjunction with a defined cell separation medium to isolate specific cell types (Section 5.1.A); (2) the use of a single-step precise density gradient medium to isolate particular cell types (Section 5.1.B); and (3) the use of carrier particles coupled to specific binding agents to enhance the efficiency of the cell separation processes of the invention (Section 5.2). In addition, isolation of exemplified cell types using the methods of the invention are described in Section 5.3.

### 5.1.A. Cell-trap Centrifuge Apparatus

In a preferred embodiment, the present invention includes a centrifuge apparatus and its use for density separation of selected cell types. For the purpose of the present invention, the term "cell-trap tube" refers to a centrifugation tube which forms part of the apparatus, and which includes constriction that forms a "trap" or fluid receiving region in the bottom portion of the tube. As will be illustrated by the description and drawings of the specific embodiments that follow, an important feature of the constriction is that it is positioned within the tube in a manner such that fluid is retained in the bottom portion of the tube below the constriction member, when the tube is inverted. The apparatus of the invention also includes cell separation material for density separation of cells.

A preferred embodiment of the centrifugation apparatus of the invention is shown in cross-section in FIGS. 1A and B. As shown, tube 10 includes constriction member 12, which defines central opening 14. Constriction member 12 preferably defines one or more downwardly sloped upper surfaces having lower edge regions defining constricted opening 14.

The bottom surface of the constriction member may also may be similarly, slightly angled (although not shown as such in the figures). In an exemplary embodiment, with a tube having an inner diameter of about 2.8 cm, the diameter of opening 14 formed by constriction member 12 is preferably about 0.5 cm. The size of opening 14 is generally not so small as to prevent heavier components of a sample, layered on top of the density gradient solution, from passing through the opening prior to actual centrifugation. Such a movement of components may occur due to normal gravitational forces. In general, the diameter of opening 14 is dictated by the ability to form an increased surface tension across the opening. A restriction that is little more than a rim around the interior of the barrel may be sufficient to provide such a surface tension. Hence, the cross-sectional area of the aperture formed by the constriction member may be as little as about 5% or as great as about 95% of the horizontal cross-sectional surface area of the tube.

While the constriction member illustrated in FIGS. 1A-D and 2A-B are annular (e.g., ring-like), it is appreciated that the constriction member may form a number of opening shapes, or may form a plurality of openings. The shape of opening 14 is not limited to a circular shape, though in general, a funnel-shaped constriction member forming a roughly circular shape will be preferred. The opening may also be oval, rectangular, star-shaped, or any other shape that would create a restricted passage within the tube, provided that the geometry provides for creation of sufficient surface tension to impede discharge of fluid in the bottom of the tube upon inversion, as described above. In addition, the constriction member may comprise a mesh or a sieve spanning the horizontal cross-section of the tube. In this case, the annular member is also said to comprise a plurality of openings.

In addition, while the opening will preferably be centered horizontally within the tube, the opening may also be off-center and achieve substantially the same results. With respect to vertical construction and positioning in the tube, the constriction member may be formed integrally with the tube or may be constructed for a forced fit. For example, an annular constriction member may be formed from an elastomeric silicone material to be inserted into the tube by a forced fit at any position along the length of the tube.

Referring again to FIG. 1A, tube 10 is filled with cell separation density gradient solution 16 to a level above constriction member 12, or, minimally, at least above opening 14. Preferably, with reference to a standard 50 ml centrifugation tube, density gradient solution 16 is filled to a level at least about 1mm above the constriction member. The fluid sample 18 to be separated is layered on the top of density gradient solution 16, and the tube and its contents are subjected to centrifugation. Preferably, the sample is carefully layered so that at least about 1 mm of density gradient solution remains between the sample and the top of the constriction member after layering.

Referring to FIG. 1B, following centrifugation, components having densities greater than that of the gradient solution are found in a pellet 20 at the bottom of tube 10. Cellular components having densities less than that of the density gradient solution 16 remain floating at the top of the solution, in an interface 22 between the gradient solution and the remaining portion of the fluid sample solution. The interface portion is then removed. Such removal can be by decantation of the tube, as indicated by arrow 24 in FIG. 1C. The provision of the density gradient solution to a level above the opening as described above helps to prevent the formation of an interface portion below constriction member 12.

Without ascribing to any particular underlying theory of the operability of the tube, it is noted that constriction member 12 facilitates pouring off the upper portion by providing a support or nucleus for formation of an intermediate surface tension across the surface of opening 14, when the tube is tilted for pouring. This surface tension impedes mixing of upper and lower portions of the tube when the contents of the upper portion are poured out of the tube. Constriction member 12 may be provided as an insert placed into a straight-walled tube. Alternatively, constriction member 12 may be formed as constriction of the tube wall during a molding process in the making of the tube itself. When the constriction member is provided by an insert, the insert may be movable to enable the operator to change the relative volumes of the lower portion 26 and upper portion 28 of tube 10 according to experimental conditions. The position of the constriction member in a molded tube can also be varied, during the manufacturing process, to provide tubes of differing relative upper and lower portion volumes. For example, in the isolation of cells from peripheral blood, a 20 ml sample of blood requires lower portion 26 to be about 15 ml in order to accommodate the relatively large amount of red blood cells centrifuged out. By comparison, a 20 ml sample of apheresis or buffy-coat blood would require only about 10 ml in the lower portion.

In many applications, it will be desirable to collect only the supernatant fraction containing the interface portion. In such cases, the pellet is discarded with the tube. In other cases, the pellet can be removed by mechanical manipulation/disruption. For example, the tube can be inverted and subjected to vortex mixing. Such mixing will disrupt the pellet into the adjacent liquid phase and will induce movement of this liquid phase and disrupted cells from the lower or collection portion of the tube into the upper portion of the tube.

An advantage of the present invention is that the low density material above the constriction member is separated from material beneath by the simple act of pouring or decanting the contents of the upper portion of the tube. This contrasts with many conventional methods of unloading gradient separations using standard straight-wall centrifuge tubes, where materials are separated by carefully pipetting out of the tube or, alternatively, by puncturing the bottom of the tube and allowing the contents of the tube to slowly drip out into collection vessels. Thus, the present invention provides a convenient, simple means for unloading differentially separated materials.

Generally, in separating cells by density centrifugation using the tube of the invention, solution, such as solution 18 in FIG. 1A containing cell mixture loaded onto the density gradient medium or material in the tube, will have a specific density that is less than the specific density of the density gradient or cell separation medium in the tube. During centrifugation, cells having a specific density that is less than or equal to the density of the cell separation

medium will settle in the interface formed between the loading solution and the density gradient material.

Another advantage of the tube of the present invention is that, unlike conventional straight-wall tubes, if the tube is dropped or accidentally inverted, the contents of the separated upper and lower portions will not readily mix, due to the presence of the constriction member. Moreover, once separation has taken place, the solution present above the constriction member can be mixed in the tube, without disturbing (or fear of contamination by) the contents of the tube below the constriction member.

In an alternative preferred embodiment, tube 10 may be provided with insert or shield 30, as shown in FIGS. 2A and 2B. Shield 30 is provided above constriction member 12 to facilitate layering of the sample onto the gradient solution. Shield 30 may take the form of a roughly concentric insert placed in the upper portion of the tube and extending at least partially around the tube. In use, the operator pipettes material between shield 30 and the tube wall. The shield directs the material along the side of the tube to the top of the density gradient solution, while minimizing disturbance of the solution. As shown in FIG. 2B, tube 10 is a clear plastic or glass, with constriction member 12 formed as a separate silicone insert. Shield 30 can be held in the upper portion of the tube, for example, by interference fit with spacers 31 biasing against the tube wall. Alternatively, shield 30 could be formed as a part of the tube.

The separation of materials may be further enhanced by the addition of valve 40 to the constriction member, as shown in FIG. 3. The valve 40 is located across opening 14. Valve 40 may be a one-way valve, or a valve that only opens upon application of a threshold centrifugal force. The valve can be formed by providing flaps of a softer material over the opening. In a preferred embodiment, the force required to open valve 40 would be about 850 times the normal force of gravity. Valve 40 thus allows heavy cells to pass through during initial centrifugation, and then keeps those cells in place, allowing for further processing of the lighter cells of interest located above the valve (such as washing or mixing of the cells). In this way complete and final manipulation of the cells can be performed in a single sterile container.

FIGS. 4A-F are illustrations of alternative shapes and designs for the tube and constriction member according to the invention. FIG. 4A shows alternative tube 42 having a separate bottom compartment 44 for receiving the pellet to provide optimal collection of cells. Constriction member 12 is as previously described; it is funnel shaped on its upper surface and formed from a separate insert of plastic or, preferably, silicone. FIG. 4B shows a tube 46 with a pointed bottom wall. Tube 46 with the pointed bottom wall also enhances cell collection by allowing the heavier cells to form a better pellet, which may be desired if the cells are to be collected. Constriction member 48 is again an insert, but with a flat upper surface and wider opening. FIG. 4C illustrates alternative tube 50 with an integrally molded constriction member 52. FIG. 4D shows an alternative constriction member 54 that facilitate movement within tube 55 to adjust the relative volumes of the upper and lower portions. For this reason constriction member 54 has annular extendings contact points 56. The constriction member will only contact the tube at these points, which create a fluid tight seal, but allow for easier adjustability. Tube 55 also has a flat bottom. FIG. 4E illustrates a further alternative embodiment of the present invention, wherein tube 60 includes cell trapping material 62, such as a sponge or gel. Material 62 may contain compounds that specifically bind certain cell types or toxins that kill specific cell types. Material 62 also may be made of a magnetic material if desired. Tube 64, shown in FIG. 4F, illustrates a further example of an integrally formed constriction member 66 in a tube with a flat bottom wall 68. Construction member 66 is located such that lower portion 26 has a smaller relative volume.

FIGS. 5A and 5B illustrate further alternative embodiments of the tube according to the invention. In each, two constriction members are provided. Second constriction member 12A is located above first constriction member 12B to create more compartments to allow separation of cells of differing densities. In FIG. 5A, the constriction members are shown as separate inserts, whereas they are integrally formed with the tube in FIG. 5B. Additional constriction members could also be added if a sample of several different densities is to be separated.

FIG. 6 depicts a centrifuge apparatus of the invention incorporated into a closed system. Such a system is particularly useful for sterile processing of samples, such as, for example, blood samples or samples that are to be later infused into a patient. As illustrated, closed system 70 includes a reservoir 71, shown as sterile bag, containing blood 72 previously collected by known techniques, is connected by sterile connecting tubing 74 to centrifuge tube 76, illustrated as a "bucket" style centrifuge tube having a closed top 77. The closed top will have at least one, and preferably at least two entry ports, useful for introduction and removal of sample, and for venting, as described below. In the embodiment shown, solid ridge 79 protruding upward from closed top 77 is included to form a protective barrier for the entry ports, and as an attachment point for a protective, removable lid for the apparatus that serves to reduce potential contamination during shipping and storage.

With further reference to FIG. 6, tubing 74 is attached to tube 76 through entry port 78, adapted with fitting 80, which may be any type of locking tip adapted for sterile connection, for example, a Luer-Lock™ syringe connector. Alternatively, fitting 80 may be a sterile septum adapted for connection with sterile fluid bags and tubes, for example a SAFSITE™ small wire extension set with reflux valve and Spin-Lock™ adaptor available from Burron Medical Inc., Bethlehem, Pennsylvania. To facilitate fluid flow into centrifuge tube 76, the apparatus contains air vent entry port 82. As shown, air filter 84 is attached to entry port 82 to prevent contamination.

In accordance with the invention, tube 76 is includes constriction member 88, formed as previously described; it is funnel shaped on its upper surface. As illustrated, constriction member 88 is formed integrally with tube 76 forming an indentation 89 on the outer surface of the tube. The constriction member is supported by supports 90 to prevent compression during centrifugation. The bucket also contains density material 91 disposed in the tube both above and below constriction member 88.

An added feature of tube 76 is entry port 92. This entry port communicates via closed fluid channel 94 with the bottom portion 95 of tube 76. The entry port and channel are used to fill the lower portion of the tube 95, for example, with density gradient cell separation medium. Alternatively, the port and channel may be used to remove materials, including cell pellet materials, from the bottom of the tube following centrifugation. This feature of the tube is neither required by nor restricted to the closed system context in which it is illustrated.

Fluid flow from reservoir 70 into tube 76 can be initiated by applying suction on air vent entry port 82, or it may be initiated by other means known in the art, including gravity. The rate of flow is adjusted, either by altering the pressure head between the two containers or by regulating a valve positioned in the tube or at the entry port at a point between reservoir 70 and entry port 78. Flow rate is optimally regulated to fill or partially fill the upper portion of tube 76, above the level of the density gradient solution. When sufficient fluid sample has entered the tube, flow can be terminated by any of a number of means known in the art, such as regulation by a valve or by lowering of pressure head. The tubing is then removed from the bucket, port 78 is sealed, and the bucket is subjected to centrifugation as described above.

In another preferred embodiment, the cell-trap tube may be used in the form of a centifugable syringe, such as centrifuge syringe 110 illustrated in Figure 7. As illustrated, centrifuge syringe 110 includes a specimen container 114 with a central orifice surrounded by fitting 112 adapted for receiving a needle 113, a handle 116 and a plunger 118. Fitting 112 may be any type of locking tip adapted to hold a needle, for example, a Luer-Lock™ syringe tip. Alternatively, fitting 112 may be a sterile septum adapted for connection with sterile fluid bags and tubes, for example a SAFSITE™ small wire extension set with reflux valve and Spin-Lock™ adaptor available from Burron Medical Inc., Bethlehem, Pennsylvania.

Handle 116 further preferably comprises knob 122 and a removable connection 124 to plunger 118. As shown, plunger 118 is single piece, machined or molded from a plastic material. The plunger preferably has a funnel-shaped bottom wall 126 that is removably connected to the handle at connection 124. Side wall 127 preferably closely matches the container wall to permit sliding movement but still provide an essentially fluid-tight barrier therearound. A top wall is formed by constriction member 128, which defines central opening 129. Alternatively, the outer diameter of side wall 127 may be slightly undersized to facilitate sliding and an o-ring seal provided between side wall 127 and container 114. Removable connection 124 may take the form of, for example, a screw fitting or a snap-fit. Preferably, connection 24 also provides for reattachment of handle 116.

The plunger 118 is filled with a density gradient material 120 before the introduction of a specimen. Preferably, the density gradient material is filled to a level above the constriction member, or at least above the top of opening 129. For example, when using a standard 50 ml syringe, having an inner diameter of about 2.8 cm, the gradient material is preferably filled to a level about 1mm or more above constriction member 128, so that the interface forms above constriction member 128.

5.1.B. <u>Density Gradient Materials and Preparation</u>

According to an important aspect of the invention, it has been discovered that significant enrichment of specific cells can be effected by centrifugation of a cell mixture on a single layer cell separation medium having a well defined specific density. The defined specific density is roughly the density of the desired cell type to be isolated. Importantly, the cell separation medium must be prepared to an accuracy of ±0.0005 gr/ml, and preferably ±0.0002 gr/ml, of the specific density of the desired specific cell type. Using such well defined medium, specific cell types can be isolated by centrifugation on a single density medium in a conventional centrifuge tube. However, as shown herein, use of a cell-trap tube in conjunction with such medium will generally facilitate improved handling as well as yield.

The apparatus and methods of cell separation of the present invention include use of a cell separation material, such as a density gradient material, having a specific gravity between 1.0000 gr/ml and 2.0000 gr/ml, preferably between 1.0300 gr/ml and 1.2000 gr/ml, that is accurate within ± 0.0005 gr/ml, preferably ± 0.0002 gr/ml of the specific gravity of the desired cell. A variety of commercially available gradient materials may be used to achieve cell isolation based on the defined density of the desired cell population, including, but not limited to, "PERCOLL", available from Pharmacia; "FICOLL HYPAQUE"; any sugar solution, *e.g.* sucrose; dextran; any protein solution, *e.g.* bovine serum albumin (BSA); iodinated low molecular weight compounds such as Metrizamide and heavy salts, *e.g.* cesium chloride.

According to an important feature of the invention, the density gradient solution should be prepared and adjusted to a pre-determined density; osmolality, most preferably in the range of 280 to 320 mOsm/kg $H_2O$; and pH, preferably from 6.8 to 7.8, and most preferably pH 7.4, for maintaining a physiologically isotonic density gradient, prior

to use. The osmolality and pH may vary depending upon the particular conditions under which the density gradient separation method is performed. For example, the temperature at which the samples are maintained or centrifuged may necessitate modifications to the osmolality and/or pH of the density gradient material, in order to maintain the appropriate density. Such modifications of the osmolality and pH will be apparent to those skilled in the art.

Cell samples should be processed within a relatively short time after collection, because the density of the cells may change according to culture, collection or storage conditions. In order to maintain the optimal isolation of desired cells from body fluids, it is preferred that blood samples are used within 48 hours after collection. Most preferably, body fluids should be subjected to density gradient centrifugation within several hours after collection. The adjusted gradient solution should be added to a centrifugation tube in a volume sufficient to allow all the cells overlaid on it to separate during centrifugation. For example, a volume of about 20-25 ml of the solution is generally adequate for separating desired cells in 20 ml of whole blood.

"SILAN PERCOLL" (S-Percoll; Pharmacia Fine Chemicals, Piscataway, NJ), classified as a colloidal silica, is one preferred density gradient material. S-Percoll is prepared from colloidal silica by reacting and thus blocking the silanol groups with an alkyl trimethoxy silane reagent and has the structural formula:

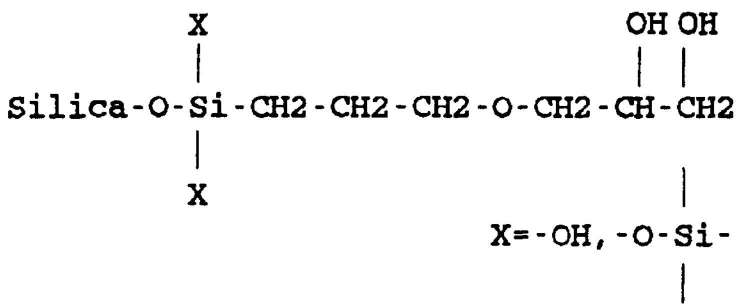

Related colloidal silicas and methods for production thereof are disclosed in U.S. Patent 4,927,749 to Dorn. A "PERCOLL" stock solution having osmolality of 280-320 mOs/kg $H_2O$ can be made by adding 12 parts of "PERCOLL" with 1 part 10x Ca and Mg free PBS or 1.5 M NaCl, for human cells, or adding 9 parts of "PERCOLL" with 1 part 10x Ca and Mg free PBS or 1.5 M NaCl, for non-human animal cells.

In carrying out the methods of cell separation of the present invention, it is generally preferred that the specific density of the cell separation material be within about $\pm$0.0005 gr/ml, preferably $\pm$ 0.0002 gr/ml of the specific density of the cells to be collected. The specific density of the stock solution up to the fourth digit may be determined using appropriate equipment, for example, a high precision digital density meter such as DMA 48 (Anton PAAR USA, Ashland, VA) which measures density with an accuracy of $\pm$0.0002 gr/ml. This aspect of invention in discussed further in Section 5.3, below.

The osmolality of the stock solution may be adjusted appropriately, for example, to 280 mOsm/kg $H_2O \pm 10$ for human use or 320 mOsm/kg $H_2O \pm 10$ for animal use. The pH may be adjusted appropriately, preferentially to 7.4 if a physiologically isotonic solution is desired.

An appropriate amount of cell separation material, prepared as described above, is placed in a centrifuge tube which is preferably, but not necessarily, a cell-trap tube. The cell mixture to be separated is layered on the tube, and the tube is subjected to centrifugation at low speed - generally about 500-1500 x g. Cells having densities greater than the specific density of the cell separation material move to the pellet portion of the tube, while those having densities less than that of the material remain at the interface between the cell diluent and the material. Generally, the method of the invention is directed to collecting the lighter fraction of cells present at the interface.

5.2. <u>DENSITY ADJUSTED CELL SEPARATION</u>

According to a further aspect of the invention the density separation methods described herein can be augmented by adding to the cell mixture to be separated, microparticle carriers to which are attached cell specific binding molecules effective to bind selected cells in the mixture. In a preferred method, such binding molecules will be used remove from the mixture during centrifugation contaminating cells having densities approximately equal to or lighter than the cells of interest. This process, referred to herein as "Density Adjusted Cell Separation" (DACS) is described in the sections that follow.

5.2.A. Isolation of Cells using Density Adjusted Cell Sorting (DACS)

FIGS. 8 (A-D) compare density adjusted cell sorting (FIGS. 8C and 8D) to conventional density gradient centrifugation (FIGS 8A and 8B). In both methods, solution 130 containing undesired cells 132 and desired cells 134 is layered on a density gradient material 131. With reference to FIG. 8B, following centrifugation, with conventional gradient centrifugation, there are still a relatively large number of undesired cell types 132 trapped at the interface with the cells of interest 134 (FIG. 8B).

Using DACS, affinity modified carrier particles are added to the cell mixture to bind to undesired cells. As shown in FIG 8C carriers 136 bind to undesired cells to form density adjusted cells 138. These cells are rendered denser and thus able to sediment during centrifugation (FIG. 8D). Alternatively, for cells which are heavier than the gradient density, a lighter density may be imparted to them by use of lighter carrier particles, *e.g.* highly porous silica particles which are rendered cell type-specific by the direct or indirect binding of cell type-specific binding agents.

When carrier particles specific for unwanted cells are mixed with a cell or tissue sample which is then overlaid onto a customized density gradient according to the methods of the invention described herein, a single centrifugation step allows for substantial enrichment of a cell type of interest from the sample. The foregoing is an example of density adjusted cell sorting applied in a negative affinity manner, *i.e.,* to purge undesired cells, *e.g.* tumor cells, from a sample by using a modified carrier particle having specificity for the undesired cell population. Alternatively, density adjusted cell sorting may applied in a positive affinity manner, *i.e.,* to collect desired cells from a sample by using a modified carrier particle having specificity for the desired cell population. The beads may be removed from desired cells by enzymatic or chemical cleavage. For example, papain may be used to cleave a desired cell from immunoglobulins. After the first round of centrifugation and collection of enriched cells, a second round of centrifugation including density adjusted cell sorting may be performed to further enrich for the desired cell population. It can be further appreciated that another advantage of DACS is that it makes somewhat less critical the precise matching of specific densities of cells of interest and the cell separation material discussed above, since unwanted cells having similar densities can be efficiently eliminated by this procedure.

Studies carried out in support of the present invention, discussed in Section 5.3.A and detailed in Example 1E, demonstrate that when complete blood from pregnant females was incubated with carrier particle-coated-anti-CD45 most leukocytes were depleted from the sample.

Further studies detailed in Example 2 demonstrate that, for isolation of $CD34^+$ cells, apheresed blood from cancer patients can be directly incubated with carrier particle-coated-anti-CD45 antibodies can also provide desired leukocyte depletion.

It can be appreciated that a variety of cell type-specific binding agents may be used to target specific cell types in the blood. The selection of such binding agents will be apparent to skilled practitioners, based on the type of cell to be collected and the cell composition of the cell mixture. Useful agents include antibodies such as the leukocyte-specific antibodies, e.g., anti-CD3, anti-CD4, anti-CD5 and anti-CD8 specific for T cells; anti-CD12, anti-CD19 and anti-CD20 specific for B cells; anti-CD14 specific for monocytes; anti-CD16 and anti-CD56 specific for natural killer cells; and anti-CD41 for platelets. Many of these antibodies are commercially available in a form already conjugated to various types of particles (AMAC, DYNAL). In addition, cell type-specific binding agents include lectins such as wheat germ agglutinin and soy bean agglutinin, growth factors and cytokines.

Alternatively, with reference to the method of isolation of hematopoietic stem $CD34^+$ cells described herein, a positive selection procedure may also be used to cause the cells to be denser, so that they are pelleted during centrifugation. In this case, antibodies directed to CD34 coated on carrier particles are used to pellet all remaining $CD34^+$ cells. Furthermore, antibodies directed to any cell surface marker may be directly linked to heavy particles for use in density adjusted cell sorting, following conjugation methods well known in the art. It is noteworthy that when density adjusted cell sorting is applied, the specific density of the gradient is less critical, as long as the undesired cells are all rendered heavier.

5.2.B. Carrier Particles

A number of commercially available carrier particles may be used in the present invention and include, for example, organic polymers, *e.g.* polyethylene; polypropylene; polyvinyl compounds *e.g.* polyvinylchloride, polyacrylonitrile, polyacrylate, polymethacrylate, polycarbonate and copolymers thereof; polystyrene latex; nylon; polyterephthlate; and the like, or inorganic polymers, *e.g.* glass or silica particles; cellulose, Dextrans, polysaccharides, *e.g.* agarose, cellulose, Sepharose, Sephadex, etc., or combinations thereof. The carrier particles may be from naturally occurring polymers, modified naturally occurring polymers and synthetic addition and condensation polymers. A preferred carrier particle of the present invention is a silica particle between 0.1-5.0 μm coupled to an aminopropyl group and having a density of greater than 1.08 gr/ml. U.S. Pat. Nos. 4,927,750 and 4,927,749, issued May 22, 1990, describe examples of modified silanes which may be used in the present invention as carrier particles. Various carrier particles

are commercially available from, for example, Bangs Laboratories, Inc., Carmel, IN., Pharmacia, Sigma Chemical Company, Bio-Rad, AMAC, Inc., etc.

A preferred heavy carrier particle of the present invention is a silica bead having a density greater than the density of the density gradient material selected according to methods described *infra* and a particle size of 0.1 μm to 5.0 μm such that the carrier particles will be pelleted upon centrifugation. Such a particle may additionally be silanized, as detailed in Example 4B herein. The preferred particle will also have the capability of binding, either directly or indirectly, to cell type-specific binding agents, or may be derivatized to such agents.

A preferred lighter carrier particle useful in the present invention is one having a density less than 1.0 and a particle size of 0.1 to 5.0 μm, such that the particles will float above the density gradient upon centrifugation as well as one having the capability of binding, either directly or indirectly to cell type-specific binding agents. Such low density carrier particles can be obtained from 3M, St. Paul MN catalog no. H50/1000, termed "Scotchlite microbeads".

Polystyrene latex particles (available from Sigma Chemical Company), may also be used as low density particles; however, due to surface hydrophobicity, such particles characteristically exhibit non-specific binding. Such non-specific binding may be reduced by treatment methods, such as pre-absorption with protein or addition of methacrylic acid groups, known in the art. Latex particles may be transformed into high density particles by addition of a metal group.

Also available for use in the invention are polyacrylamide carrier particles, such as Bio-Rad polyacrylamide beads coupled to either primary amino functional groups (Affi-Gel 701 beads) or carboxyl functional groups (Affi-Gel 702 beads or Immunobead Reagent).

The preparation of small, stable, spherical particles which are bio-compatible, *i.e.*, do not interact non-specifically with cells or other biological components and which contain functional groups to which specific proteins and other bio-chemical molecules can be covalently bonded, is disclosed in U.S. Pat. No. 3,957,741, issued May 19, 1976. The hydroxyl or amino groups can be activated by cyanogen bromide for covalent bonding of protein and other chemicals containing amino groups to the polystyrene latex.

U.S. Pat. No. 4,035,316, issued July 12, 1977, describes the method for making cell specific, variable density, polymeric microspheres derivatized with amine-, hydroxyl- and/or carboxyl-functional groups and having a density of at least 1.30 gr/ml preferably above 1.40 gr/ml or a density below 1.15 gr/ml and preferably below 1.08 gr/ml.

A process for forming highly uniform silica spheres is described in U.S. Pat No. 4,983,369, issued Jan. 8, 1991.

Also included within the scope of the present invention are carrier particles or carrier particles to which are attached a magnetic substance allowing for enhanced separation of cells through use of a magnetic field. U.S. Pat. No. 4,777,145, issued October 11, 1988 describes an immunological assay method using magnetic particles. AMAC, Inc., subsidiary of IMMUNOTECH, S.A. (France) provides magnetic microspheres coated with monoclonal antibody, *e.g.* anti-CD4, anti-CD8, anti-CD19, anti-45, etc.

5.2.C. Cell Type-Specific Binding Agents

The cell separation methods of the present invention may include the use of cell type-specific binding agents having specificity for either the desired or undesired cell populations which may be bound either directly or indirectly to a carrier particle, as described in the previous section. As defined herein, "cell type-specific binding agents" include, for example, antibodies or antigens; peptides or polypeptides; growth factors or cytokines; lectins and agglutinins or other affinity molecules known in the art to have specificity for either the desired cell populations or the undesired cell populations. Many of these agents are commercially available or can be produced according to well known methods.

Commercially available cell type-specific binding agents that may be used in the present invention, include, but are not limited to, antibodies, antigens, polypeptides, peptides, growth factors, membrane bound receptors, small organic molecules, lectins, and agglutinins.

A variety of antibodies known to those of skill in the art, commercially available or available through cell culture depositories, e.g. the ATCC, Rockville, Md. or the NRLL, Peoria, II., may be used in the present invention as cell type-specific binding agents depending upon the cell type desired to be isolated or enriched. These include, but are not limited to, antibodies specific to hematopoietic and lymphoid antigens such as, anti-CD2, anti-CD2R, anti-CD3, anti-CD4, anti-CD5 and anti-CD8 specific for T cells, anti-CDw17 specific for granulocytes, monocytes and platelets, anti-CD18 specific for leucocytes, anti-CD71 specific for activated T- and B-cells, macrophages, proliferating cells, antibodies to cytokines and growth factors *(e.g.* IL1-IL13, EGF, IGF I and II, TGF-α and β, TNF-α and β, FGF, NGF, CIF, IFN-α and β, CSF's), hormones, cellular or tumor associated antigens or markers, adhesion molecules, hemostasis molecules, and endothelial cells. The precise agents to be used will depend upon the antigenicity of the desired cell and the composition of the cell mixture from which it is to be isolated. Skilled practitioners will be able to determine optimal carrier agent composition, based on these factors.

Antibodies useful as affinity molecules can be produced according to standard polyclonal or monoclonal production methods known in the art. Useful antibodies include, but are not limited to, polyclonal, monoclonal, chimeric,

single chain, Fab fragments and fragments produced by a Fab expression library and phage display expression library.

Commercially available agglutinins may be used in the present invention. These include, but not limited to, wheat germ agglutinin, peanut agglutinin, soy bean agglutinin, phytohaemagglutinin, and leucoagglutinin, and are commercially available, for example, from Pharmacia Fine Chemicals (Piscataway, NJ).

5.2.D. Coupling of Cell Type-Specific Binding Agents to Carrier Particle

Immobilization of the cell type-specific binding agent to the carrier particles can be achieved by a variety of techniques known to those of skill in the art. Such techniques are described in, for example Bangs (The Latex Course (1992), available from Bangs Laboratories, Inc. Carmel, IN); Yoshioka *et al.,* 1991, Journal of Chromatography 566: 361-368; Pope *et al.,* 1993, Bioconjugate Chem. 4:166-171); Harlow and Lane, 1988, Antibodies: A Laboratory Manual, Colorado Spring Harbor Laboratory; Avidin-Biotin Chemistry: A Handbook, 1992, ed. Savage *et al.,* pub. PIERCE; Hermanson *et al.,* Immobilized Affinity Ligand Techniques, 1992, pub. Academic Press, Inc.

Binding techniques include, for example, simple physical absorption or adsorption where the cell type-specific binding agent is bound directly to the carrier protein without the use of functional groups; complex adsorption where a second binding agent, *e.g.* BSA, is co-adsorbed to the carrier particle and forms the basis for binding functional groups; and covalent bonding of the binding agent to the carrier particle. The biotin-strepavidin affinity system may also be used in the present invention to bind cell type-specific binding agents to the carrier particles. Various particle surface chemical reactions for covalent coupling are known to those of skill in the art and include, but not limited to, carboxylic acid, primary or aliphatic amine, aromatic amine or aniline, chloromethyl (vinyl benzyl chloride), amide, aldehyde, hydroxyl, thio, hydrazide, epoxy, sulfate and sulfonate. Other coupling chemical reactions are described in Bangs, Uniform Latex Particles (1984).

For use in the present invention, it is preferred that the direct or indirect binding of the cell type-specific binding agent to the carrier particle be performed in excess binding agent to allow for maximum coverage of the surface of the carrier particle, thereby reducing the potential for non-specific binding and bead clumping. Carrier particles may also be subjected to blocking agents, *e.g.* casein, gelatin and TWEEN detergent to fill any unoccupied sites on the carrier particle in order to reduce non-specific binding.

In one illustrative example, carboxyl groups on the carrier particle surface can be made reactive with the available amino groups on the cell type-specific binding agent. Other means of binding cell type-specific binding agent to particle surfaces include employing activated carboxylic acids, carbodiimides, *i.e.,* (1-ethyl-3-(3-dimethylaminopropyl) carbodiimide or EDAC, imido esters, active alkyl halides, etc., to form amido, amidine or amino linkages.

A preferred carrier particle of the present invention is an aminopropyl silica particle wherein the amino groups have been coupled to the silica particle through a glutaraldehyde linkage, *see* Example 4.

5.3. ISOLATING SELECTED CELL TYPES

The present invention provides methods for isolation or enrichment of a desired cell type from an *in vivo* or *in vitro* mixture, when the specific density of the cell type is known. Exemplary specific cell types isolated by this method are discussed below.

Preferably, the method includes loading one of the embodiments of the cell-trap cell separation apparatus described in Section 5.1.A., with a cell separation medium having a specific density that is within ±0.0005 gr/ml of the specific density of the, then centrifuging the apparatus. The desired cells are then collected from the upper portion of the centrifugation tube.

While use of the cell-trap centrifugation apparatus aids in the convenience and efficiency of the method of the invention, it should be noted that the invention is based, in part, on the specificity and accuracy of the density gradients used. That is, the density gradients must be prepared such that their densities are accurate to within at least ±0.0005 gr/ml, and preferably within ±0.0002 gr/ml, of the specific density of the desired cell, at a defined osmolality. Thus the invention may be practiced using a standard centrifugation tube, as well as a cell-trap tube, so long as the density is well defined. Examples 1-3 herein describe use of specific density cell separation media with and without the cell-trap.

Following centrifugation, the desired cell will be found at the interface between the gradient material and the cell sample solution. Material at the interface can be collected, then centrifuged to concentrate it, if desired. Alternatively, when it is preferable to retain the desired cell material in a closed system, the centrifugation can be carried out in a closed system or in a centrifugation syringe having a cell-trap configuration, such as described in Section 5.1.A.

If desired, improved purity of the desired cell type can be achieved by adding to the above procedure a density-adjusted cell sorting procedure, as described in Section 5.2. As described therein, prior to centrifugation, the cell sample containing the desired cell is exposed to carrier particles having bound thereto cell type-specific binding agents effective to bind and remove unwanted cells.

For therapeutic applications, it is preferred that the desired cell population remain in the interface between the gradient material and the cell sample solution. Alternatively, if the desired cells are attached to beads in the pellet, they may be cleaved enzymatically or chemically from the beads by methods known to those of skill in the art, including the use of proteolytic enzymes, *e.g.* papain.

Cells isolated or enriched by the cell separation methods described herein may be used for a variety of diagnostic and therapeutic uses. The isolated or enriched cells may be cultured under sterile conditions and subjected to cytogenetic analysis, *e.g.* the detection of chromosomal abnormalities and gender determination. The isolated or enriched cells may be reacted with molecular probes for more sensitive detection using PCR and FISH. The isolated or enriched cells may also be used therapeutically, for example, for allogeneic and autologous transplantation.

The following sections provide specific guidance for isolation of three therapeutically and/or diagnostically useful cell types that are isolated in accordance with the principles of the present invention.

5.3.A Isolation of Fetal Cells from Maternal Blood

It has now been established that a small number of fetal cells circulate in maternal blood, including lymphocytes, trophoblasts, and nucleated red blood cells. These cells provide an alternative source of genetic materials for prenatal testing (Simpson and Elias, 1993, JAMA 270:2357). However, the rarity of such cells in maternal blood (estimated 1 in $10^7$-$10^8$ maternal blood cells) dictates the need for enhanced methods of isolation and detection (Holzgreve *et al.,* 1992, J. Reprod. Med. 37:410). While several detection methods have been made available through recent advances, including polymerase chain reaction (PCR) and fluorescence in situ hydribization (FISH), the major difficulty in the routine use of maternal blood for prenatal diagnosis is the inability to enrich the small number of fetal cells in a mixture of maternal cells to yield reliable diagnostic results.

Several techniques have been proposed to meet this need, including fluorescence-activated cell sorting (Herzenberg *et al.,* 1979, Proc. Natl. Aca. Sci. USA 76:1453), magnetic-activated cell sorting (Ganshirt-Ahlert *et al.,* 1992, Am. J. Obstet. Gynecol. 166:1350) or a combination of these procedures (Ganshirt-Ahlert *et al.,* 1992, Am. J. Hum. Genet. *51*:A48). While these procedures have been able to partially enrich fetal cells, they are both costly, because of the need for sophisticated instrumentation, and cumbersome, due to multiple steps involved. More importantly, such methods result in substantial cell loss, thereby reducing the number of fetal cells available for subsequent analysis.

Of the fetal cell types known to be present in maternal blood during pregnancy, nucleated red blood cells are the most attractive candidates for enrichment and detection. The nuclei of these cells provide a source of genetic material for the application of techniques such as PCR and FISH. There are also several well-known commercially available antibodies that may be used to characterize the cells, such as anti-CD71 and anti-glycophorin A. Another major advantage for targeting fetal cells in the erythroid lineage is their relatively short life span. Unlike lymphocytes, these cells should not persist from prior pregnancies to interfere with the analysis of maternal blood.

The apparatus and methods of the invention can be used to isolate fetal cells from cell mixtures. In particular, the method can be used to isolate rare fetal cells from maternal blood for purposes of pre-natal diagnosis by genetic testing.

This aspect of the invention is based, in part, on Applicant's discovery that colloidal silica (PERCOLL) solution adjusted to a density of 1.0720 ± 0.0005 gr/ml, an osmolality of 280 mOsm/kg $H_2O$, and pH 7.4 efficiently separates fetal cells from the majority of adult blood cells when complete blood without prior separation or dilution is overlaid on the gradient solution. In addition, the method is improved by using cell-trap centrifugation tubes described herein which contain a constriction to allow the cells in the upper portion *(i.e.,* above the constriction) to be decanted as opposed to using a pipette to collect the cells which results in increased cell loss.

In practice, peripheral blood may be collected from pregnant females in anti-coagulant-containing tubes or by apheresis or leukopheresis. Complete blood does not need to be processed or diluted prior to centrifugation. However, since the methods enrich fetal cells based on their specific buoyant density, it is important that the cells are subject to separation within a relatively short time after collection, because the density of the cells changes according to their culture or storage conditions. Therefore, in order to obtain optimal enrichment of fetal cells from maternal blood, it is preferred that the blood samples are used within 48 hours after their collection. Most preferably, blood samples should be subjected to density gradient centrifugation within several hours of collection.

The present invention may be practiced by overlaying whole blood on a defined density gradient in a cell-trap centrifugation tube. After centrifugation the cells in the interface are collected and pelleted by another centrifugation step to remove cell debris, platelets, and the remainder of the density material. Thereafter, the cells are depleted of the majority of leukocytes by their reactivity with an antibody, such as anti-CD45; the remaining red blood cells analyzed by flow cytometry, and the fetal cells within this population identified by FISH using specific molecular probes.

The efficiency of the method is can be further improved when it is combined with density adjusted cell sorting, described in Section 5.2, above. Thus, this specific embodiment of the invention provides for a rapid and high yield procedure to enrich for fetal cells from a large blood volume. The increased number of fetal cells in the resultant cell

population enhances the sensitivity and accuracy of techniques commonly applied to genetic testing.

In an effort to further minimize cell loss, but achieve platelet removal, a second centrifugation step for pelleting the fetal nucleated red blood cells may also be carried out in a cell-trap tube.

Example 1 details materials and methods for isolating nucleated fetal cells according to the methods of the invention. Table 1 presents results from an experiment in which "PERCOLL" was used as the density gradient material in a one-step gradient separation according to the present invention. "PERCOLL" was prepared and adjusted to physiologic osmolality of 280 mOsm/kg $H_2O$ and physiologic pH of 7.4, as described in Example 1.

Summarizing the results of the experiments detailed in Example 1, the specific density of the cell separation medium was adjusted as described. Cells were harvested from the interface, and percent fetal and pellet and interface and tested for yield by marker antigen characterization. As summarized in Table 1, a density of 1.0720 gr/ml or above produced at least about 50% recovery of nucleated cells from the total nucleated cell population prior to centrifugation, there was a substantial contamination of the interface with mature red blood cells when the gradient was adjusted to a density of 1.0750 gr/ml or above.

From the foregoing it will be appreciated that in order to recover a high percentage of total nucleated cells from the starting cell mixture, but reduce mature red blood cell contamination a density of 1.0720 gr/ml is optimal. Moreover, this density should be defined with a precision ± 0.0005 gr/ml, and preferably, of ± 0.0002 gr/ml.

TABLE 1

| Density of "PERCOLL" (gr/ml) | Percentage of Nucleated Cells Recovered from Interface After Centrifugation | Percentage of Mature Red Blood Cell Contamination |
|---|---|---|
| 1.0820 | 60% | 21% |
| 1.0790 | 58% | 21% |
| 1.0770 | 56% | 25% |
| 1.0750 | 45% | 20% |
| 1.0720 | 50% | 2% |
| 1.0700 | 21% | 2.2% |
| 1.0640 | 25% | 2.1% |
| 1.0610 | 23% | 1.7% |

When four cell separation methods were compared for nucleated red blood cell yield, the two methods of the present invention produced substantially higher percentages of nucleated red blood cells than the conventional method. Table 2 shows results of experiments in which cells were isolated by four different methods. In all but the DACS treated sample, centrifuged samples were depleted with anti-CD45 antibodies coupled to magnetic beads, according to methods known in the art. From these results, it can be seen that cell-trap tubes containing "PERCOLL" at a density of 1.0720 ± 0.0002 gr/ml produced about a 20 fold higher number of nucleated red blood cells than the conventional method using stock "FICOLL" at a density of 1.077 ± 0.001 gr/ml and 320 mOsm/kg $H_20$. Thus, this method resulted in a statistically significant increase the total number of fetal nucleated red blood cells available for subsequent genetic testing, compared to conventional methods. In addition, the procedure also enriched for fetal trophoblast cells. Further inspection revealed fetal cells at different stages of differentiation present in the enriched fractions. Furthermore, the DACS method produced comparable results to the method requiring magnetic field depletion of $CD45^+$ undesired cell populations.

Any tubes suitable for use in centrifugation may be used for the practice of the invention. In a preferred embodiment, the present invention is directed to a cell-trap tube for the density separation of fetal cells. For the purpose of the present invention, a cell-trap tube refers to a centrifugation tube which contains within it a constriction or a trap and a properly adjusted density gradient material filled to a level above the constriction so that cells having a certain density pass through the opening of the constriction to form a cell pellet in the compartment below the constriction during centrifugation.

**TABLE 2**

| | Cell Number Before Separation | Percentage of Nucleated Cells Recovered from Interface | | Percentage of Nucleated Red Blood Cells | Percentage of Nucleated Fetal Red Blood Cells |
|---|---|---|---|---|---|
| | | After Density Centrifugation | After Anti-CD45 Depletion | | |
| Conventional Method Using "FICOLL" | $10^7$ | 14% | 0.2% | 0.01% | Undetectable |
| "FICOLL" plus Cell-trap | $10^7$ | 16% | 0.17% | 0.01% | 0.05% |
| "PERCOLL" Plus Cell-trap | $10^7$ | 54% | 1.7% | 0.21% | 0.41% |
| "PERCOLL" Plus Cell-trap Plus Density Adjusted Cell Sorting | $10^7$ | 3.3% | Not Applicable | 0.23% | Not Done |

In the foregoing experiments, DACS treatment of the maternal samples used anti-CD45 as an agent to deplete unwanted blood cells from the interface of the density gradient. A variety of other cell type-specific binding agents may be used to target specific cell types in the blood. These agents encompass antibodies such as anti-CD3, anti-CD4, anti-CD5 and anti-CD8 specific for T cells; anti-CD12, anti-CD19 and anti-CD20 specific for B cells; anti-CD14 specific for monocytes; anti-CD16 and anti-CD56 specific for natural killer cells; and anti-CD41 for platelets. Many of these antibodies are commercially available in a form already conjugated to various types of particles (AMAC, DYNAL). Alternatively, for positive selection of nucleated red blood cells, mature red blood cells may be removed from the sample and antibodies directed to glycophorin A or CD71 coated on carrier particles may be used to pellet all remaining nucleated red blood cells.

The nucleated red blood cells enriched by the methods described above were subsequently examined for the presence of fetal cells. The enriched cell preparations obtained from donors who were known to be carrying a male fetus were selected for use in FISH analysis. The cells were incubated with an X-chromosome-specific probe linked to a green fluorescence dye and a Y-chromosome-specific probe linked to a red fluorescence dye. Fetal nucleated red blood cells were identified as cells with nuclei that contained a red spot and a green spot under a fluorescence microscope, while other cells were of maternal origin. The far right column of Table 2 shows that there was an eight fold increase in the number of XY (fetal) chromosomes in the cell populations prepared by one method of the invention over that by the conventional method. It is of further interest to note that the method of using cell-trap and "FICOLL"

also increased the number of fetal nucleated red blood cells to the detection threshold over the same gradient practiced without cell-trap tubes, indicating that the cell-trap itself was useful in increasing cell yield.

It should be noted that in order to obtain reliable diagnostic results involving techniques such as FISH, it is generally necessary to enrich the fetal cells to at least 0.1% of the final cell preparation in order for the enrichment method to be used as a routine procedure. The method of the invention is shown herein to have clearly exceeded this limit by demonstrating enrichment of fetal cells to a level of 41 cells/10,000 total cells analyzed.

5.3.B Isolation of Hematopoietic Progenitor $CD34^+$ Cells

Transplantation of bone marrow and peripheral blood is performed in the clinic for the treatment of cancer and hematopoietic disorders. Hematopoietic progenitor cells migrate to and reconstitute the bone marrow microenvironment after transfusion. Depending on their degree of differentiation these progenitor cells either contribute to short term engraftment or to long term engraftment of the bone marrow microenvironment.

In general, there are two major types of cells that are considered to be necessary for successful transplantation and engraftment - colony forming unit cells (CFU), that provide short term bone marrow engraftment preventing infection in the patient during the time immediately following the radio- and chemotherapy, and long term culture initiating cells (LTC-IC), that establish a long lasting, self-renewing myeloid and lymphoid system in the patient. The hematopoietic progenitor cell population expressing the CD34 surface antigen contains both CFU and LTC-IC. Hence, the present invention relates to methods for enriching total $CD34^+$ cells.

In addition, when the bone marrow cells or other progenitor cell source contain contaminating tumor cells that must be purged prior to re-infusion in an autologous setting, the large number of total cells with a low percentage of $CD34^+$ cells makes it technically difficult to perform adequate purging of tumor cells. Thus, there remains a need for a simple method for enriching $CD34^+$ progenitor cells from a cell mixture containing higher numbers of these cells that are amenable to efficient purging of residual tumor cells for use in subsequent transplantation.

In an effort to address these problems, investigators have focused on the use of anti-CD34 antibodies. Such procedures involve positive selection, such as the passage of white blood cells over a column containing anti-CD34 antibodies or binding of cells to magnetic bead-conjugated anti-CD34 antibodies or by panning on anti-CD34-coated plates, and collecting the bound cells. However, the affinity based methods have limitations in terms of practical utility in that they are costly and time-consuming.

Alternative methods for enriching hematopoietic progenitor cells have been reported which utilize various forms of density gradient centrifugation (Olofsson et al., 1980, Scan. J. Haematol. 24:254; Ellis et al., 1984, J. Immunol. Meth. 66:9; Lasky and Zanjani, 1985, Exp. Hematol. 13:680; Martin et al., 1986, Brit. J. Haematol. 63:187). However, all reported methods use agar colony assays to identify hematopoietic progenitor cells after enrichment. It is known that the progenitor assays only detect committed precursor cells which occupy less than 1% of the $CD34^+$ population. It is therefore uncertain whether these methods can in fact enrich for the early progenitor cells or stem cells which can permanently engraft and reconstitute a lymphohematopoietic system, as they have not been tested clinically. Furthermore, there is no indication from the published reports that any of these procedures are able to obtain adequate numbers of cells for clinical use.

The present invention provides methods of rapid and high yield enrichment of progenitor cells based on density gradient centrifugation. More specifically, the invention utilizes a precisely determined density of a density gradient solution, preferably contained within a specially designed cell-trap centrifugation tube to maximize cell yield.

A major advantage of the methods described herein is that a large volume of apheresed blood may be directly placed on the density gradient. Peripheral blood may be collected in anti-coagulant-containing tubes or by apheresis or leukopheresis. In addition, the single step process reduces the total volume of infusate by 70-90%, thereby reducing the amount of cryopreservative required. After enrichment, the final cell preparation represents between 10% to 30% of the starting cell number, but contains between 70% and 100% of the starting number of $CD34^+$ cells and colony-forming CFU's. Due to this high yield (70% to 100%) of $CD34^+$ cells, a single peripheral blood collection may yield sufficient $CD34^+$ cells to reconstitute the hematopoietic and immune system of patients undergoing ablative chemotherapy. This cell population also contains a reduced number of T cells, but a substantial number of natural killer cells and natural suppressor cells. Additionally, the procedure is rapid, convenient and cost effective. Processing of a complete sample requires no specialized instrumentation and can be performed by one person in a time frame of one hour.

The present invention also provides for purging from the bone marrow cells or other progenitor cell source contaminating tumor cells, prior to re-infusion of the progenitor cells in an autologous setting for use in subsequent transplantation.

For purposes of purging, the density of a given tumor cell is determined by centrifuging a tumor containing sample on a discontinuous density gradient ranging from 1.0490 to 1.0640 gr/ml. The majority of undesirable, non-tumor cells represent cells of the immune and hematopoietic system and have a density in the range of 1.0610 to 1.0770. The density of tumor cells generally falls within the 1.0490 to 1.0580 gr/ml density range. The density of the

cell separation medium is determined to an accuracy of within ± 0.0005 gr/ml, preferably ± 0.0002 gr/ml of the specific gravity of the desired progenitor cells. Thus tumor purging can be accomplished by a two step process, in which the progenitor cells are first pelleted through a cell separation medium capable of retaining tumor cells. Alternatively, the DACS technique can be used to remove tumor cells in a one-step process, according to the methods described herein.

For the enrichment of $CD34^+$ cells, the cell separation medium should be adjusted to a density of 1.0605 ± 0.0005 gr/ml, a physiologic osmolality of 270-290 mOsm/kg $H_20$ and physiologic pH 6.8-7.8. More preferably, the density will be 1.0605 ± 0.0002 gr/ml, and the osmolality will be 280 mOsm/kg $H_2O$, at pH 7.4.

In an illustrative example, apheresed blood from a cancer patient treated with G-CSF is directly loaded into a cell-trap centrifugation tube containing a "PERCOLL" solution filled to a level above the constriction, which has been adjusted to the preferred density of 1.0605 ± 0.0005 gr/ml, osmolality of 280 mOsm/kg $H_20$ and pH7.4. The density of the "PERCOLL" solution may be adjusted on a densitometer to precisely define its accuracy up to at least the fourth decimal place. It should be noted that a variety of other gradient materials may be used to achieve progenitor cell enrichment, and they include, but are not limited to, "FICOLL", "FICOLL-HYPAQUE", cesium chloride, any protein solution such as albumin or any sugar solution such as sucrose and dextran. However, the density gradient solution should be prepared and adjusted to the appropriate density, osmolality and pH according to that disclosed herein, prior to its use. The gradient solution should be added to a centrifugation tube in a volume sufficient to allow all the cells having a higher density to pass through the gradient during centrifugation. For example, a volume of about 20-25 ml of the solution is generally adequate for separating cells in 20 ml of apheresed blood samples.

Any tubes suitable for use in centrifugation may be used for the practice of the invention. In a preferred embodiment of the present invention, the cell-trap centrifugation apparatus described in Section 5.1.A. will be used for the density separation of $CD34^+$ cells.

Table 3 presents results from studies in which "PERCOLL" was used as the density gradient material. "PERCOLL" was prepared and adjusted to physiologic osmolality of 280± 10 mOsm/kg $H_2O$ and physiologic pH of 7.4. For this study, the starting cell mixture was a sample of apheresed blood from a non-Hodgkin lymphoma patient who had been treated with G-CSF. When the gradient was adjusted to different densities, the results showed that when the density was at 1.0600 gr/ml or above, there was an about 60-90% increase of $CD34^+$ cells in the interface fraction over the gradients adjusted to lower densities. Furthermore, the percentage of total cell yield also increased slightly at 1.0600 gr/ml or above. Thus, in order to recover a high percentage of total $CD34^+$ cells from the starting cell mixture, the density of 1.0605 gr/ml was chosen. It was further determined that an accuracy of within ±0.0005 gr/ml was preferable to ensure high yield enrichment of $CD34^+$ cells.

In additional experiments carried out in support of the present invention, "PERCOLL" was adjusted to a density of 1.0605 gr/ml and osmolality of 280 mOsm/kg $H_2O$, and compared with stock "FICOLL" which had a density of 1.077 ± 0.001 gr/ml and 320 mOsm/kg $H_2O$. Table 4 shows that when the gravitational force of centrifugation increased, more $CD34^+$ cells were pelleted in the stock "FICOLL" gradient. Since the use of unadjusted "FICOLL" was the standard material used for density gradient separation of $CD34^+$ cells from a cell mixture, these results show that a precisely defined density range could substantially enhance the high yield enrichment of $CD34^+$ cells from a cell mixture. As shown in Table 4, the percentage of $CD34^+$ cell yield after centrifugation at 1500 x g increased about 2 fold over that achieved by a conventional method.

**TABLE 3**

| Density "PERCOLL" (gr/ml) | Percentage of Total Cell Yield | Percentage of $CD34^+$ Cell Yield |
|---|---|---|
| Unfractionated | 100% | 100% |
| 1.0590 Interface<br>Pellet | 11%<br>85% | 32%<br>68% |
| 1.0595 I<br>P | 18%<br>78% | 45%<br>55% |
| 1.0600 I<br>P | 26%<br>70% | 80%<br>20% |
| 1.0605 I<br>P | 31%<br>63% | 83%<br>17% |
| 1.0610 I<br>P | 35%<br>60% | 89%<br>11% |

**TABLE 4**

| Gravitational Force (xg) | "FICOLL" | | "PERCOLL" | |
|---|---|---|---|---|
| | Percentage of $CD34^+$ Cell Purity | Percentage of $CD34^+$ Cell Yield | Percentage of $CD34^+$ Cell Purity | Percentage of $CD34^+$ Cell Yield |
| 200 | 0.62% | 1% (Baseline) | 0.83% | 0.64% |
| 350 | 0.63% | 1.07% | 0.85% | 0.49% |
| 800 | 0.74% | 0.6% | 1.92% | 0.88% |
| 1500 | 0.62% | 0.48% | 2.05% | 0.83% |

Absolute cell numbers and cell recovery were determined using apheresed blood samples from non-Hodgkin lymphoma patients. The mean cell recovery from 5 samples was variable but was always in the range of 90%. Since cell counting was performed after a washing step, that may account for cell loss up to 10%. $CD34^+$ cell recovery was determined from the 5 different blood samples, and was always in the range of 90%. This result was similar to the non-specific cell loss shown above, thus it was not due to a specific depletion of the total number of $CD34^+$ cells or a change in the CD34 expression by hematopoietic progenitor cells. When the quantitative recovery of CFU's was determined, the recovery of CFU was also in the range of 90%. Therefore, the cell separation procedure by the 1.0605 gr/ml density gradient did not change the functional potential of hematopoietic progenitor cells to form colonies.

In addition, the quantitative distribution of the CFU over the gradient was determined. The results shown in FIG. 10 demonstrate that only minor numbers of CFU were observed in the pellet fractions and approximately 90-100% of the CFU were present in the interface of 1.0605 gr/ml "PERCOLL". Also, it was observed that 100% of the CFU-GEMM were present in the interface (FIG. 11). LTC-IC assays showed that between 90-100% of the uncommitted hematopoietic stem cells were present in the interface (FIG. 12).

Hence, these data demonstrate that the centrifugation of apheresed blood on a single-layer gradient adjusted to 1.0605 ± 0.0005 gr/ml resulted in a minor non-specific loss (10% or less) of the total cell product. However, while

the interface represented approximately 30% of the total cell number, this cell population contained 70-90% of the $CD34^+$ cells and more than 90% of the CFU's. The interface contained 100% of the CFU-GEMM, and since CFU-GEMM represented progenitor cells with a low degree of hematopoietic commitment and a limited degree of self renewal, the interface also contained the uncommitted hematopoietic stem cells. The results obtained with the LTC-IC assays further support this conclusion. This simplified procedure may allow the automation of $CD34^+$ cell enrichment in a completely closed system. Furthermore, experiments performed in cell-trap tubes produced similar results with an even greater degree of consistency.

Additional indications for $CD34^+$ cells. Graft versus host disease (GvHD) is induced by the T-cells that are present in the donor allografts. Consequently, some transplant protocols have included the total removal of T-cells from the graft prior to transplantation. Although these methods successfully reduced GvHD, they also resulted in increased incidence of graft failure and tumor relapse. In other words, the presence of a limited number of T-cells may be beneficial for the survival chances of allotrans-plant patients. In this context, a "PERCOLL" gradient was adjusted to a density of 1.0605 ± 0.0005 gr/ml to test for its ability to remove T-cells. Normal bone marrow and apheresed blood samples from G-CSF treated normal individuals were processed on the density gradient. The cells from the interface and pellet fractions were stained with the T-cell specific anti-CD3 antibodies. FIG. 13 shows that for both tissue sources the interface contained between 10% and 20% of the total number of T-cells that were present in the unprocessed material.

*In vitro* studies have shown that human bone marrow contains low density cells that block *in vitro* alloresponses in the mixed lymphocyte reactions (MLR). Based on the fact that this suppressive activity was HLA non-restricted, it was referred to in the literature as natural suppressor (NS) activity. A "PERCOLL" density gradient was adjusted to a density of 1.0605 ± 0.0005 gr/ml to test for its ability to enrich cells with NS activity. Apheresed blood samples from lymphoma patients and from normal individuals that received G-CSF treatment were centrifuged on a discontinuous five layer gradient, and the interfaces and pellet were screened for their potential to suppress the mixed lymphocyte culture. FIG. 14 shows that cells with NS activity had a density equal or lighter than 1.0605 gr/ml. Consequently, more than 90% of the NS activity was present in the final cell preparation after centrifugation on a 1.0605 gr/ml gradient.

NK cells have been shown to kill autologous tumor cells. From a clinical perspective, it may be beneficial to have increased numbers of NK cells in the transplant to reduce tumor relapse. In this context, the density of the NK cells was determined on a discontinuous five-layer "PERCOLL" gradient. NK cells also showed a density equal to or lighter than 1.0605 gr/ml. Consequently, more than 90% of NK cells was present in the final cell preparation after centrifugation on a 1.0605 gr/ml gradient, as shown in FIG. 15.

FIG. 16(A-F) shows the result of a representative experiment in which $CD34^+$ cells were enriched using the DACS procedure in conjunction with the density method of the invention, by removing contaminating cells with an anti-CD45 mAb coupled to a heavy carrier (such as magnetic beads or aminopropyl glass beads). In this particular experiment the total cell number was reduced 82% and the CD34 yield was around 40%. CD34 purity was increased from 2% to approximately 20%. Since the anti-CD45 antibody removed also some of the $CD34^+$ cells, this method could improved by using a mixture of other antibodies to deplete non-stem cells.

5.3.C. Enrichment of Breast Tumor Cells

It is established that breast tumor cells and tumor emboli spread directly to the bloodstream providing an alternative and desirable source of breast tumor cells for diagnostic purposes (Cecil Textbook of Medicine, *supra).* However, in order to successfully utilize circulating bodily fluids for breast cancer diagnosis, the small number of circulating breast tumor cells must first be enriched, and one must employ highly sensitive and specific techniques to detect the breast tumor cells.

At present, there is a need for a rapid and reproducible procedure suitable for processing a large volume of whole blood which produces high-yield, specific enrichment of breast tumor cells from circulating bodily fluids. This can be accomplished by application of the methods of the present invention to detection of metastatic breast tumor cells from such fluids.

The present invention relates to methods of rapid and high yield isolation or enrichment of breast tumor cell populations from cell sources or cell mixtures, based on density gradient centrifugation. More specifically, the present invention relates to the use of a specially designed cell-trap centrifugation tube containing a precisely determined density of a density gradient solution and a manner of collecting the desired cell population which maximizes yield. The method of the present invention may be used to increase the sensitivity of breast tumor detection by enriching the tumor cells from a cell source, *e.g.* whole blood, prior to the use of molecular or cellular detection techniques. In combination with tumor-specific antibodies and density adjusted cell sorting, this method allows the purging of tumor cells from a bone marrow or peripheral blood stem cell graft.

The present invention demonstrates that proper adjustments of a gradient material to a specific density, osmolality and pH greatly enhance cell separation. For the enrichment of breast tumor cells, a gradient should be adjusted to a density of 1.0490 to 1.0580 ± 0.0005 gr/ml, depending upon the specific type of breast tumor cell, a physiologic

osmolality of 270-290 mOsm/kg $H_20$ and physiologic pH 6.8-7.8. In a specific embodiment by way of examples, breast tumor cells are directly loaded into a cell-trap centrifugation tube containing an appropriate cell separation medium, such as "PERCOLL" solution filled to a level above the constriction. In the specific examples described herein, successful enrichment of tumor cells was carried out on "PERCOLL" density gradient material which had been adjusted to the specific density of between 1.0490 to 1.0580 ± 0.0002 gr/ml, depending upon the specific type of breast tumor cell, osmolality of 280 mOsm/kg $H_20$ and pH 7.4. The density of the "PERCOLL" solution may be adjusted on a densitometer to precisely define its accuracy, as described in Section 5.1.B.

The cells enriched by the methods described in Example 3 may subsequently be examined for the presence of breast tumor cells. The resultant yield of isolated or enriched cells from the cell separation methods of the present invention may be used for diagnostic purposes, *e.g.* morphological, molecular, biochemical or immunophenotypic assays. For example, DNA may be prepared from the collected cells and subjected to polymerase chain reaction (PCR) or the collected cells may be assessed morphologically thereby avoiding the use of invasive and expensive surgical procedures heretofore relied upon for such a determination.

Various breast tumor antigens and breast tumor markers are known to those of skill in the art or are commercially available including but not limited to cathepsin D (Westley *et al.,* 1980), EGF-R (Osborne *et al.,* 1980), estrogen receptor (Gorski *et al.,* 1966), Ki-67 (Gerdes *et al.,* 1983), progesterone receptor (Horowitz *et al.,* 1983), and TGF-$\alpha$, associated with breast cancer. Antibodies directed to these antigens or markers may be used to assess the tumor type of collected cells.

A major advantage of the methods described herein is that a large volume of complete blood may be directly placed on the density gradient. Peripheral blood may be collected in anti-coagulant-containing tubes or by apheresis or leukopheresis. Complete blood does not need to be processed or diluted prior to centrifugation. However, since the methods enrich breast tumor cells based on their specific buoyant density, it is important that the cells are subject to separation within a relatively short time after their collection from an *in vivo* source because the density of the cells changes according to their culture or storage conditions. Therefore, in order to obtain optimal enrichment of breast tumor cells from blood, it is preferred that the blood samples are used within 48 hours after their collection. Most preferably, blood samples should be subjected to density gradient centrifugation within several hours of collection.

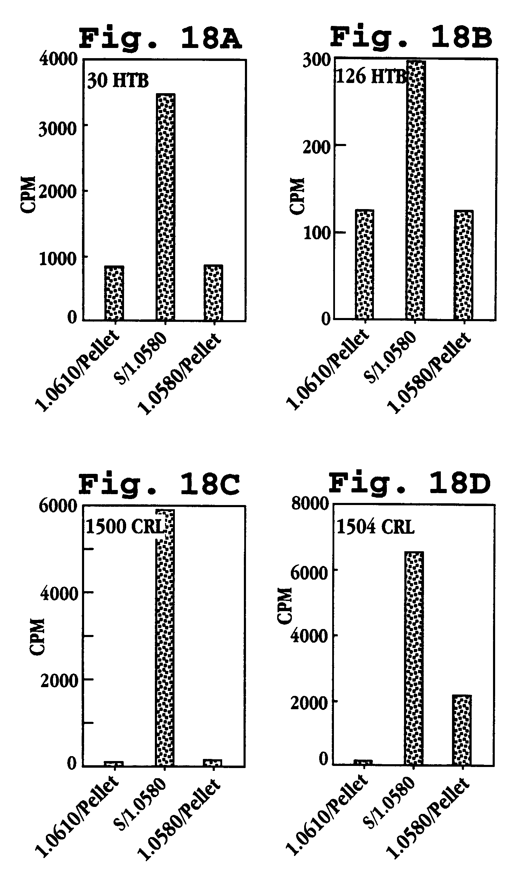

The densities of four breast tumor cell types, derived from tumor cell lines, were determined using "PERCOLL" discontinuous density gradient system (Figures 17A-17D). The cells were collected from each of the interfaces and counted in a hemocytometer. The results showed that 30 to 60% of the tumor cells have a density equal to or higher than 1.0580 g/ml (Figure 18A-18D). This implies that the fraction containing tumor cells was between 10 and 80% pure. Of the cells collected in a specific density of 1.0580, approximately 75 to 85% of the total cells were tumor cells, while approximately 10% of the total cell volume was a contaminant. This implies that the detection limit of the assay is improved approximately 10 times from $1/10^6$ to $1/10^5$.

When radioactively labeled breast tumor cells were mixed with a peripheral blood buffy coat, up to 80% of them could be retained by centrifuging the mixture on a 1.0580 gr/ml, 280 mOsm gradient. In addition, only a small fraction (<10% of initial cell number) of non-tumor cells contaminated the collected tumor fraction.

The density ranges shown in Figures 17A-17D and 18A-18D, obtained from using cultured breast tumor cells, are likely to be applicable to breast tumor cells obtained from *in vivo* sources. It will be apparent to those of skill in the art that slight variations in the densities of various breast tumor cells from *in vivo* sources may necessitate refinement of the exact density necessary to achieve optimum enrichment. Methods for determining specific densities with an accuracy of ± 0.0002 gr/ml are disclosed herein.

The foregoing method may also be used in conjunction with the DACS procedure described herein. For example, complete blood can be directly incubated with carrier particle- coated-anti-CD45 antibodies which react with most leukocytes. Since breast tumor cells do not react with anti-CD45 to any significant degree, the vast majority of the non-red blood cells, leukocytes, and other cells are rendered heavier than the density material and pellet during centrifugation, while the breast tumor cells remain in the upper compartment. A variety of other cell type-specific binding agents may be used to target specific cell types in the blood, as discussed in the previous sections.

Alternatively, the positive selection DACS procedure may be used to cause the breast tumor cells to be heavier than their normal density so that they are pelleted during centrifugation. Cell type-specific binding agents useful in the positive selection procedure include, but are not limited to antibodies to breast tumor antigens and antibodies to breast tumor markers, *e.g.* CA 15-3 (Kufe *et al.,* 1984), CA 549 (Bray *et al.,* 1987), cathepsin D (Westley *et al.,* 1980), EGF-R (Osborne *et al.,* 1980), estrogen receptor (Gorski *et al.,* 1966), Ki-67 (Gerdes *et al.,* 1983), progesterone receptor (Horowitz *et al.,* 1983), and TGF-$\alpha$, associated with breast cancer. Many of these antibodies are commercially available.

The cells enriched by the methods described herein can subsequently be examined for the presence of breast tumor cells. The resultant yield of isolated or enriched cells from the cell separation methods of the present invention may be used for diagnostic purposes, *e.g.* morphological, molecular, biochemical or immunophenotypic assays. For example, DNA may be prepared from the collected cells and subjected to polymerase chain reaction (PCR) or the collected cells may be assessed morphologically thereby avoiding the use of invasive and expensive surgical procedures heretofore relied upon for such a determination. Alternatively, tumor cells can be identified by the presence of tumor cell markers known to those of skill in the art, including but not limited to cathepsin D (Westley *et al.,* 1980), EGF-R (Osborne *et al.,* 1980), estrogen receptor (Gorski *et al.,* 1966), Ki-67 (Gerdes *et al.,* 1983), progesterone receptor (Horowitz *et* al., 1983), and TGF-α, associated with breast cancer. Antibodies directed to these antigens or markers may be used to assess the tumor type of collected cells.

## 6. EXAMPLES

### EXAMPLE 1

### ISOLATION OF NUCLEATED RED BLOOD CELLS FROM MATERNAL BLOOD

#### A. PREPARATION OF DENSITY GRADIENTS

"PERCOLL" solution was purchased from Pharmacia Biotech (Uppsala, Sweden) and stored at 4°C according to the recommendation of the vendor. A stock solution was prepared by mixing 12 parts of "PERCOLL" with 1 part of 10 x calcium and magnesium-free phosphate buffered saline (PBS). The pH of the solution was adjusted to 7.4 and the osmolality to 280 ± 10 mOsm/Kg $H_2O$. For use in separating fetal nucleated red blood cells in a blood sample, the stock solution was further diluted with calcium and magnesium-free PBS to a density of 1.0720 ± 0.0005 gr/ml and used at room temperature. It was important to adjust the density of the gradient to an accuracy within ± 0.0005 gr/ml, preferably within 0.0002 gr/ml of 1.0720 gr/ml in order to ensure reproducibility and accuracy of cell separation. This was done by a high precision digital density meter such as DMA 48 (Anton PAAR USA, Ashland, VA). All procedures were performed under sterile conditions and at room temperature.

#### B. COLLECTION AND PROCESSING OF BLOOD SAMPLES

Peripheral blood was collected from pregnant females in anti-coagulant-containing tubes. The collection was performed before week 20 of pregnancy because the number of fetal cells circulating in maternal blood generally began to decline after week 17 of pregnancy. An optimal collection time is week 13. After collection, the blood samples were processed within 48 hours.

#### C.1. DENSITY GRADIENT CENTRIFUGATION OF PERIPHERAL BLOOD

Complete blood samples were layered on a "PERCOLL" cell separation medium previously adjusted to a density of 1.0720 ± 0.0002 gr/ml, an osmolality of 280 mOsm/Kg $H_2O$, and a pH of 7.4 in a 50 ml conical cell-trap tube. The tube contained a constriction in a location so that approximately 15ml of "PERCOLL" was in the lower compartment and 5ml of "PERCOLL" was above the constriction. Generally, 20 ml of blood samples were layered on top of this gradient. The tube was centrifuged at 850 x g for 30 minutes at room temperature. The cells lodged at the interface of the gradient; *i.e.,* on top of "PERCOLL," were collected by pouring the entire content of the upper compartment of the tube into another 50 ml tube. The cell pellet and associated liquid in the region below the constriction remained therein when the tube was inverted. After centrifugation at 650 x g for 10 minutes at room temperature, the fluid on top of the pellet was removed with a pipette, and the cells in the pellet resuspended in PBS. Since this low speed centrifugation step was primarily used to concentrate the cells of interest into a pellet, while removing cell debris and platelets in the upper region, a cell-trap could also be used to facilitate this step. In this alternative embodiment, a modified 50ml cell-trap tube was used in which the constriction was placed near the bottom of the tube so that a small volume of about 0.5ml was below it. This design would protect the pellet and reduce cell loss during removal of the fluid above the pellet after centrifugation. This specific feature would also allow the method of the invention to be used in an automated fashion without the need for a subsequent cell sorting step, which was performed to reduce contaminating cells, particularly platelets.

The cell separation method described above was compared with conventional methods, and the following procedure was also carried out as a control. This procedure was similar to previously published methods known in the art (Bianchi *et al.,* 1990, Proc. Natl. Acad. Sci. USA 87:3279).

The blood sample was collected as described above and diluted 1:4 in PBS. The diluted blood was layered on "FICOLL-HYPAQUE" (Pharmacia) in 4 different 15 ml tubes. The density of the stock "FICOLL" solution was at 1.077± 0.001 gr/ml and the osmolality at 320 mOsm/kg $H_2O$ as published by Pharmacia. The tubes were centrifuged at 850 x g for 20 minutes at room temperature. The cells at the interface above the "FICOLL" were collected with a pipette and transferred to two 15 ml tubes. The tubes were filled to the top with PBS and spun at 650 x g for 10 minutes at room temperature. The fluid on top of the pellet was aspirated with a pipette, and the pellet resuspended in PBS. In

addition, experiments were also performed using cell-trap tubes with "FICOLL" solution in an effort to increase cell yield.

C.2 AFFINITY SEPARATION BY A MAGNETIC FIELD

The fetal nucleated red blood cells resuspended in PBS after density gradient centrifugation described above were further enriched through removal of $CD45^+$ leukocytes by incubating the cells with an anti-CD45 monoclonal antibody (clone ALB-12) (Biodesign International, Kennebunk, ME) for 30 minutes at 4°C. The unbound antibodies were removed by washing the cells in PBS. A goat-anti-mouse antibody conjugated to magnetic particles (Immunocon) was added to the cells for 30 minutes at 4°C. The cells were washed in PBS and exposed to a magnetic field which attracted the magnetic particle-coated $CD45^+$ leukocytes, while the fetal nucleated erythroid and trophoblast cells remained in solution. The fetal cells were collected with a pipette and washed once in PBS. The cells were then tested by antibody staining and flow cytometric analysis to determine the number of nucleated red blood cells.

D.1 DENSITY ADJUSTED CELL SORTING

Complete blood collected from pregnant females was incubated with 1.4μ aminopropyl glass beads (Bangs Laboratories Inc., Carmel, IN) that were glutaraldehyde coated with an anti-CD45 antibody (ALB-12) for 45 minutes at room temperature. The entire blood cell mixture was layered on "PERCOLL" (1.0720 ± 0.0002 gr/ml, 280 mOsm/Kg $H_2O$, pH 7.4) in a 50 ml cell-trap tube. The tube contained about 15ml of "PERCOLL" below the constriction and about 5ml above it. It was critical to completely fill the volume under the constriction with "PERCOLL" to prevent the formation of air bubbles. The tube was centrifuged at 850 x g for 30 minutes at room temperature. The leukocyte-depleted cell population was collected from the interface above the "PERCOLL" by pouring off the entire upper region of the tube into a 50 ml tube. The cells were spun at 650 x g for 10 minutes at room temperature, and the fluid on top of the pellet removed with a pipette. The cells in the pellet were resuspended in PBS.

Alternatively, the second centrifugation step can be carried out in a modified cell-trap tube as described in Section 5.2 *supra.* Additionally, a second density adjusted cell sorting could also be performed using antibodies such as anti-CD41 to specifically remove platelets.

D.2. ANTIBODY STAINING AND FLOW CYTOMETRIC ANALYSIS

The leukocyte-depleted cell population in PBS were treated with the DNA dye LDS 751 (Exciton, Inc., Dayton, OH)and erythroid lineage specific FITC-conjugated monoclonal antibodies such as anti-glycophorin A and anti-CD71 (Becton Dickinson, Inc., San Jose, CA). The LDS 751 dye distinguished between nucleated and a nucleated cell. One million cells were incubated with 10μl antibodies for 30 minutes at 4°C in the presence of 5% rabbit serum and LDS 751. The cells were washed twice with PBS and fixed in 1% paraformaldehyde. The antibody-bound cells were analyzed by flow cytometry for which statistical analysis was performed on $10^4$ flow events using a FACSScan system equipped with a LYSYS II program.

EXAMPLE 2

ENRICHMENT OF $CD34^+$ CELLS FROM BLOOD CELL MIXTURE

A. PREPARATION OF DENSITY GRADIENTS

"PERCOLL" solution was purchased from Pharmacia Biotech (Uppsala, Sweden) and stored at 4°C according to the recommendation of the vendor. A stock solution was prepared by mixing 12 parts of "PERCOLL" with 1 part of 10 x calcium and magnesium-free phosphate buffered saline (PBS). The pH of the solution was adjusted to 7.4 and the osmolality to 280 mOsm/Kg $H_2O$. For use in separating $CD34^+$ cells in a cell mixture, the stock solution was further diluted with calcium and magnesium-free PBS to a density of 1.0605 ± 0.0005 gr/ml and used at room temperature. It was crucial to adjust the density of the gradient to an accuracy of within ± 0.0005 gr/ml of 1.0605 gr/ml in order to ensure reproducibility and accuracy of cell separation. This was done by a high precision digital density meter such as DMA 48 (Anton PAAR USA, Ashland, VA). All procedures were performed under sterile conditions and at room temperature.

B. COLLECTION OF PERIPHERAL BLOOD AND BONE MARROW

Patients were hydrated and treated with cyclophosphamide (4 $gm/m^2$) administered by intravenous (IV) infusion over two hours through a central venous catheter. Twenty-four hours after the completion of the cyclophosphamide

infusion, patients are treated with G-CSF (Neupogen, Amgen, Thousand Oaks, CA) administered by subcutaneous (SC) injection at a dose of approximately 10 µg/kg/d. Apheresis was initiated upon recovery of the white blood cell count (WBC) to equal or more than 1 x $10^9$/L. Apheresis was performed using a Cobe Spectra Cell Separator (Lakewood, Colorado) at a rate of 80 ml/mln for 200 min (total volume of 16 L).

C. DENSITY GRADIENT CENTRIFUGATION OF APHERSED BLOOD AND BONE MARROW BUFFY COATS

Apheresed peripheral blood was applied directly onto the density gradient. However, complete blood and bone marrow aspirates were processed to a buffy coat (removal of red cells) before they were applied onto the density gradient.

Apheresed blood or bone marrow buffy coat samples were layered on a "PERCOLL" gradient previously adjusted to a density of 1.0605 ± 0.0005 gr/ml, an osmolality of 280 mOsm/Kg $H_2O$, and a pH of 7.4 in a 50 ml conical cell-trap tube or a commercially available tube. The cell-trap tube contained a constriction in a location so that approximately 15ml of "PERCOLL" was in the lower compartment and 5ml of "PERCOLL" was above the constriction. It was critical to completely fill the volume under the constriction with "PERCOLL" to prevent the formation of air bubbles. Generally, 20ml of apheresed blood samples were layered on top of this gradient. The tube was centrifuged at 850 x g for 30 minutes at room temperature. The cells lodged at the interface of the gradient; *i.e.*, on top of "PERCOLL," were collected by pouring the entire content of the upper compartment of the tube into another 50 ml tube. The cell pellet in the region below the constriction were prevented from pouring off when the tube was inverted.

In order to compare the cell separation method described in the preceding paragraph with conventional methods, the test samples were also was layered on "FICOLL-HYPAQUE" (Pharmacia). The density of the stock "FICOLL" solution was at 1.077± 0.001 gr/ml and the osmolality at 320 mOsm/kg $H_2O$ as published by the vendor.

D. DENSITY ADJUSTED CELL SORTING

Apheresed blood product was incubated with 1.4µ aminopropyl glass beads (Bangs Laboratories Inc., Cannel, IN) that were glutaraldehyde coated with an anti-CD45 antibody (clone ALB-12, Biodesign International, Kennebunk, ME) for 45 minutes at room temperature. The entire blood cell mixture was layered on "PERCOLL" (1.0605 ± 0.0005 gr/ml, 280 mOsm/Kg $H_2O$, pH 7.4) in a 50 ml tube.

E. CONJUGATION OF MONOCLONAL ANTIBODIES TO CARRIER PARTICLES

Phycoerythrin-conjugated (PE) anti-CD34 monoclonal antibodies (hematopoietic progenitor cell marker) and fluorescein-conjugated (FITC) anti-CD45 monoclonal antibodies (pan-leukocyte marker) were obtained from Becton Dickinson, Inc. (San Jose, CA). Unconjugated antibodies directed to CD45, CD16 (granulocytes, monocytes), CD3 (T cells), CD14 (monocytes) were prepared in the laboratory, according to methods well known in the art.

Antibodies were conjugated to either goat anti-mouse coated magnetic beads or to goat anti-mouse coated aminopropyl glass beads by overnight incubation at room temperature. Alternatively, the antibodies could be bound directly to these beads without the goat anti-mouse bridge or could be bound via an avidin-biotin coupling reaction. In addition the antibodies could be cleaved into Fab2 fragments in order to reduce non-specific binding of cells to the beads via their Fc portion.

F. ANTIBODY STAINING AND FLOW CYTOMETRIC ANALYSIS

Cells were incubated with 10µL of an antibody and the DNA dye LDS 751 (Exciton Inc., Dayton OH) per $10^6$ cells for 30 min. on ice in the presence of 5% rabbit serum. Rabbit serum was used to reduce non-specific binding to the cells. The cells were washed twice with PBS and subsequently fixed with 1% paraformaldehyde. Statistical analysis was performed on $10^4$ flow events using a FACSScan flow cytometry system equipped with a LYSYS II program.

G. COLONY FORMING (CFU) ASSAY/FUNCTIONAL DETERMINATION OF COMMITTED $CD34^+$ CELLS

The functional characteristics of the $CD34^+$ cells in a cell sample was determined by the colony formation assay (CFU). This assay allowed the quantification of the number of committed hematopoietic progenitor cells in the cell solution. $10^5$ cells were mixed in 2mL semi-solid methyl cellulose containing different colony stimulating factors and erythropoietin (Terry Fox Laboratories, Vancouver). The entire mixture was incubated for 14 days at 37°C. The number of erythroid (CFU-E, BFU-E), granulocyte/macrophage (CFU-GM) and mixed (CFU-GEMM) colonies were counted under an inverted microscope (40x).

H. LONG TERM CULTURE INITIATING CELL (LTC-IC) ASSAY/FUNCTIONAL DETERMINATION OF UNCOMMITTED $CD34^+$ CELLS

The number of uncommitted hematopoietic progenitor cells in a cell mixture was determined by the long term culture initiating culture. The cells were seeded on an irradiated stroma feeder layer and a determination of CFU's was made in function of time. Hematopoietic stem cells were able to self-renew and gave rise to CFU's in this system for a period that exceeded 5 weeks. Long term bone marrow stromal cultures were initiated in 96 well plates ($10^6$ cells in 200 µl per well) in α-MEM medium supplemented with 12.5% horse serum, 12.5% fetal calf serum, 2 mM L-glutamine, 0.2 mM i-inositol, 20 µM folic acid, $10^{-4}$ M 2-mercaptoethanol and were kept at 33°C in a humidified atmosphere. At weekly intervals, half the medium was removed and replaced by an equal volume of fresh medium. After 2 weeks of culture, the confluent stroma layers were gamma irradiated (2000 rad) to kill endogenous hematopoietic cells. Unfractionated samples and cell preparations after separation were seeded onto the irradiated stroma layers in the same medium supplemented with $10^{-6}$M hydrocortisone. After five weeks of culture the adherent and non-adherent cells were collected and screened in the CFU assay as in Part G, above.

I. NATURAL KILLER (NK) CELL ASSAY

K562 target cells were labeled with 100 µCi $^{51}$Cr for 1 hour at 37°C and then washed four times and counted. The target cells were co-cultured for 4 hours in V-bottom 96 well multiwell plates with unfractionated apheresed blood and cells from the different fractions after cell separation. Effector and target cells were mixed at different ratios, 100:1, 50:1, 25:1 and 12:1. For example, the 100:1 ratio contained $5x10^5$ effector cells and $5x10^3$ target cells. After the incubation period, 100µl of the supernatant was harvested and counted in a scintillation counter. Maximal and spontaneous $^{51}$Cr release was measured counting either 50µl of the stock target solution and supernatant from the effectors by themselves, respectively. The percent cytotoxicity was determined according to formula:

$$\text{Percent Cytotoxicity} = \frac{\text{cpm experiment - cpm spontaneous release}}{\text{cpm maximal release - cpm spontaneous release}}$$

J. MIXED LYMPHOCYTE CULTURE AND NATURAL SUPPRESSOR CELL ACTIVITY

Cells from two different buffy coats were mixed in a flat bottom 96 well multiwell plate at $10^5$ cells of each. One of the buffy coats received 3000 rad and was referred to as the "stimulators". The other buffy coat was used untreated and referred to as "responders." Unfractionated apheresed peripheral blood products (APBL) or cells from the different density fractions were added to these co-cultures at $10^5$ cells per well. These cells were referred to as "suppressors" and received 1500 rad prior to being added to the MLR. The cells were cultured for 5 days and then pulsed with [$^3$H]-thymidine (1 µCi/well). 18 hours later, the cells were harvested and the amount of thymidine incorporated determined in a scintillation counter. The percent suppression induced by the suppressor cells was determined by the formula:

$$\text{Percent Suppression} = \frac{\text{cpm control - cpm experiment}}{\text{cpm experiment}}$$

EXAMPLE 3

DETERMINATION OF DENSITY OF BREAST TUMOR CELL DENSITY AND THEIR ENRICHMENT BY DENSITY GRADIENT CENTRIFUGATION

Cells were incubated with $^3$H thymidine for 24 hours under standard culture conditions according to methods known in the art. The cells were mixed with buffy coat from peripheral blood.

A. PREPARATION OF DENSITY GRADIENTS

"PERCOLL" solution was purchased from Pharmacia Biotech (Uppsala, Sweden) and stored at 4°C according to the recommendation of the vendor. A stock solution was prepared by mixing 12 parts of "PERCOLL" with 1 part of 10 x calcium and magnesium-free phosphate buffered saline (PBS). The pH of the solution was adjusted to 7.4 and the osmolality to 280 mOsm/Kg $H_2O$. For use in enriching breast tumor cells obtained from *in vitro* cell lines in a cell mixture, the stock solution was further diluted with calcium and magnesium-free PBS into five different fractions with respective densities of 1.0490, 1.0520, 1.0550, 1.0580, and 1.0610 and used at room temperature. It was crucial to

adjust the density of the gradient with an accuracy of ± 0.0002 gr/ml point in order to ensure reproducibility and accuracy of cell separation. This was done by a high precision digital density meter such as DMA 48 (Anton PAAR USA, Ashland, VA). All procedures were performed under sterile conditions and at room temperature.

B. DENSITY GRADIENT CENTRIFUGATION

Radioactively labeled breast cancer cells were mixed with a buffy coat from a healthy donor and centrifuged on the discontinuous gradient. Cell mixtures containing the breast tumor cells from cell lines 30 HTB, 126 HTB, 1500 CRL and 1504 CRL were layered on a "PERCOLL" gradients previously adjusted to a densities in the range of 1.0490-1.0610, ± 0.0002 gr/ml, an osmolality of 280 mOsm/Kg $H_2O$, and a pH of 7.4 in a 50 ml conical cell-trap tube. The tube contained a constriction in a location so that approximately 15 ml of "PERCOLL" was in the lower compartment and 50 ml of "PERCOLL" was above the constriction. It was critical to completely fill the volume under the constriction with "PERCOLL" to prevent the formation of air bubbles. Generally, 20 ml of cell samples were layered on top of this gradient. The tube was centrifuged at 850 x g for 30 minutes at room temperature. The cells lodged at the interface of the gradient; *i.e.,* on top of "PERCOLL," were collected by pouring the entire content of the upper compartment of the tube into another 50 ml tube. The cell pellet in the compartment below the constriction were prevented from pouring off when the tube was inverted. After centrifugation at 650 x g for 10 minutes at room temperature, the fluid on top of the pellet was removed with a pipette, and the cells in the pellet resuspended in PBS. Since this low speed centrifugation step was primarily used to concentrate the cells of interest into a pellet, while removing cell debris and platelets in the fluid, a cell-trap could also be used to facilitate this step. In this alternative embodiment, a modified 50 ml cell-trap tube was used in which the constriction was placed near the bottom of the tube so that a small volume of about 0.5 ml was below it. This design protects the pellet and reduces call loss during removal of the fluid above the pellet after centrifugation. This specific feature would allow the method of the invention to be automated without the need for cell sorting.

EXAMPLE 4

PREPARATION OF CARRIER PARTICLES

A. PREPARATION OF BEADS

Silica beads (1.4 µm) obtained from Bangs Laboratories, Carmel, IN were washed with concentrated HCl for 2 hours at room temperature and vortexed intensely every 15 minutes to break up bead clumps. After washing, the beads were centrifuged at 850 g for 5 minutes. The HCl containing supernatant was decanted and the beads were washed with deionized $H_2O$ with intensive vortexing to break up the clumps.

The beads were incubated at room temperature overnight in concentrated nitric acid with constant stirring using a magnetic stirrer. The beads were then centrifuged at 850 g for 5 minutes and washed 3 times with deionized water, using 50 ml of deionized $H_2O$ at each step. The beads were vortexed intensely in between each wash to avoid bead clumping. To prevent microbacterial contamination, the beads were stored at 0-4 degrees centigrade in deionized $H_2O$ until further use.

B. SILANIZATION OF THE BEADS

To silanize the beads, either 3-aminopropyltriethoxysilane, (3-iodopropyl)trimethoxysilane or [1-9trimethoxysilyl)-2(m-(or p) chloromethyl)phenyl] ethane were used. Forty mls of silane solution (a 10% solution in 95% ethanol/deionized $H_2O$) was added per 4 gr of beads. The bead mixture was rotated end over end for 1 hour at room temperature. The beads were centrifuged at 850 g for 5 minutes and the excess silane was washed off using 95% ethanol/deionized $H_2O$ in a volume of 100 ml. The beads were vortexed intensely in between each wash step to avoid bead clumping. After the washing step, the beads can be dried and stored. Alternatively the beads can be stored in 95% ethanol/deionized $H_2O$ in the cold which prevents clumping of the beads.

C. ANTIBODY COUPLING TO THE AMINOPROPYL GLASS

The silanized beads were incubated overnight in 2.5% glutaraldehyde at room temperature. The next day, the beads were centrifuged at 850 g for 5 minutes and the free glutaraldehyde was washed off with deionized $H_2O$ in a volume of 100 ml per 5 gr beads. The beads were vortexed intensely in between each wash step to avoid bead clumping.

The antibody was added to the aminopropyl beads in an excess, at least 3 $mg/m^2$ total bead surface and rotated end over end overnight at room temperature. The next day, the beads were centrifuged at 850 g for 5 minutes

and the free protein was washed off with 100 ml of deionized $H_2O$. The beads were vortexed intensely in between each wash step to avoid bead clumping. The beads were stored in deionized $H_2O$ containing 0.1 sodium azide in the cold. The final bead suspension should contain 70-90% single beads and 10-30% predominantly duplet and triplet beads.

The binding efficiency of the antibody conjugated beads (in terms of the percent of beads that are coated) can be determined using flow cytometric analysis and a fluoresceinated antibody directed to the coupled antibody. Alternatively, the antibody may be added to the silanized beads directly without the glutaraldehyde linking.

## Claims

**1.** A cell-separation apparatus, comprising:

a centrifugation tube having side walls and a closed bottom;
a constriction member disposed within the tube, said constriction member positioned and constructed to retain fluid in the bottom portion of the tube below the constriction member, when the tube is inverted, and
a cell-separation medium contained in the bottom portion of the tube and, prior to centrifugation of said tube, extending above said constriction member to a level above an opening formed by said constriction member, such that cells which are captured at an interface between the cell-separation medium and a lower-density medium, after centrifugation, are discharged with the lower-density medium when the tube is inverted.

**2.** The apparatus of claim 1, wherein said constriction member defines one or more downwardly sloped surfaces having lower edge regions defining a constricted opening.

**3.** The apparatus of claim 1, wherein said constriction element is an annulus having a central constricted opening.

**4.** The apparatus of claim 3, wherein said annulus is an elastomeric annulus constructed for forced fit into the tube at a selected tube level.

**5.** The apparatus of claim 1, wherein said constriction member defines a plurality of openings.

**6.** The apparatus of claim 1, wherein said tube includes a closed top. and a port in said top through which fluid material can be introduced into the tube.

**7.** The apparatus of claim 6, which further includes a second port in said top and a closed fluid channel communicating the second port with the bottom of the tube below said constriction member.

**8.** The apparatus of claim 1, for use in isolating $CD34^+$ hematopoietic progenitor cells, wherein the specific density of said cell separation medium is within ±0.0005 gr/ml of the specific density of said $CD34^+$ hematopoietic progenitor cells.

**9.** The apparatus of claim 8, wherein said cell separation medium has an osmolality of 280±10 m0sm/kg $H_2O$ and a specific density of 1.0605 gr/ml.

**10.** The apparatus of claim 1, for use in isolating nucleated fetal cells from a cell mixture also containing maternal blood cells, wherein the specific density of said cell separation medium is within ±0.0005 gr/ml of the specific density of said fetal cells.

**11.** The apparatus of claim 10, wherein the cell separation medium has an osmolality of 280±10 m0sm/kg $H_2O$ and a specific density of 1.0720 gr/ml.

**12.** The apparatus of claim 1, for use in isolating breast tumor cells from a cell mixture, wherein the cell separation medium has an osmolality of 280±10 m0sm/kg $H_2O$ and a specific density selected from the range 1.0490-1.0580 gr/ml.

**13.** A method of isolating a selected cell from a cell mixture containing one or more other cell types with densities different from that of the selected cell type, comprising

adding to a centrifugation apparatus defined by any of claims 1-7 and containing a cell-separation medium having a specific density that is within ±0.0005 gr/ml of the specific density of the selected cell type, a cell mixture containing the selected cell type,
centrifuging said apparatus at a gravitational force sufficient to pellet cells having specific densities greater than the specific density of the density gradient material in said tube, and
collecting from the upper portion of said tube of said apparatus a cell fraction containing the selected cell.

**14.** The method of claim 13, wherein said collecting is by decantation from the device of medium present in the upper portion of the device.

**15.** The method of claim 13, further comprising incubating said cell mixture with a cell type-specific binding agent linked to carrier particles prior to centrifugation, said particles having a specific density that is at least 0.001 gr/ml greater than the specific density of said cell separation medium.

**16.** The method of Claim 15, wherein said cell-type specific binding agent does not bind to the selected cell type to be isolated.

**17.** The method of claim 13, for enriching $CD34^+$ cells from a cell mixture, wherein said cell separation medium has an osmolality of 280±10 m0sm/kg $H_2O$ and a specific density of 1.0605±0.0002 gr/ml.

**18.** The method of claim 13, for use in isolating nucleated fetal cells from a cell mixture also containing maternal blood cells, wherein said cell separation medium has an osmolality of 280±10 m0sm/kg $H_2O$ and a specific density of 1.0720±0.0002 gr/ml.

**19.** The method of claim 13, for use in isolating breast tumor cells from a cell mixture, wherein said cell separation medium has an osmolality of 280±10 m0sm/kg $H_2O$ and a specific density selected from the range 1.0490-1.0580 gr/ml.

**20.** A method of isolating $CD34^+$ cells from a cell mixture, comprising

layering the cell mixture onto a cell separation medium in a centrifuge tube, said cell separation medium having a specific density of 1.0605±0.0005 gr/ml and an osmolarity of 280±10 m0sm/kg $H_2O$,
centrifuging said centrifuge tube at a gravitational force sufficient to pellet cells having a specific density of greater than the specific density of said separation medium, and
collecting said $CD34^+$ cells from the upper portion of said tube.

**21.** A method of isolating nucleated fetal cells from a cell mixture, comprising

layering the cell mixture onto a cell separation medium in a centrifuge tube, said cell separation medium having a specific density of 1.0720±0.0005 gr/ml and an osmolarity of 280±10 m0sm/kg $H_2O$,
centrifuging said centrifuge tube at a gravitational force sufficient to pellet cells having a specific density of greater than said specific density of said separation medium, and
collecting said fetal cells from the upper portion of said tube.

**22.** A method of isolating breast tumor cells from a cell mixture, comprising

layering the cell mixture onto a cell separation medium in a centrifuge tube, said cell separation medium having a specific density selected from the range 1.0490-1.0580 gr/ml and an osmolarity of 280±10 m0sm/kg $H_2O$,
centrifuging said centrifuge tube at a gravitational force sufficient to pellet cells having a specific density of greater than said specific density of said separation medium, and
collecting said breast tumor cells from the upper portion of said tube.

**23.** A method of isolating natural killer cells from a cell mixture, comprising

layering the cell mixture onto a cell separation medium in a centrifuge tube, said cell separation medium having a specific density of 1.0605±0.0005 gr/ml and an osmolarity of 280±10 m0sm/kg $H_2O$,
centrifuging said centrifuge tube at a gravitational force sufficient to pellet cells having a specific density of greater than said specific density of said separation medium, and

collecting said natural killer cells from the upper portion of said tube.

**Patentansprüche**

1. Zelltrennungsvorrichtung, umfassend:

   ein Zentrifugationsröhrchen, das Seitenwände und einen geschlossenen Boden hat;
   ein in dem Röhrchen angeordneter Verengungsteil, der so positioniert und konstruiert ist, daß Flüssigkeit im Bodenteil des Röhrchens unterhalb des Verengungsteils zurückgehalten wird, wenn das Röhrchen umgedreht wird, und
   ein Zelltrennungsmedium, das im Bodenteil des Röhrchens enthalten ist und sich, vor dem Zentrifugieren des Röhrchens, über das Verengungsteil bis zu einer Höhe über einer Öffnung, die durch das Verengungsteil gebildet wird, ausdehnt, so daß Zellen, die an einer Zwischenfläche zwischen dem Zelltrennungsmedium und einem Medium geringerer Dichte gefangen sind, nach der Zentrifugation mit dem Medium geringerer Dichte abgegeben werden, wenn das Röhrchen umgedreht wird.

2. Vorrichtung nach Anspruch 1, wobei der Verengungsteil eine oder mehrere nach unten abfallende Oberflächen aufweist, die untere Randregionen haben, die eine verengte Öffnung ausmachen.

3. Vorrichtung nach Anspruch 1, wobei das Verengungselement ein Ring ist, der eine zentrale, verengte Öffnung hat.

4. Vorrichtung nach Anspruch 3, wobei der Ring ein elastomerer Ring ist, der für einen Preßsitz im Röhrchen bei einer ausgewählten Röhrchenhöhe konstruiert ist.

5. Vorrichtung nach Anspruch 1, wobei das Verengungsteil eine Vielfalt von Öffnungen aufweist.

6. Vorrichtung nach Anspruch 1, wobei das Röhrchen einen verschlossenen oberen Teil aufweist und in diesem oberen Teil eine Durchlaßöffnung ausgebildet ist, durch welche flüssiges Material in das Röhrchen eingeführt werden kann.

7. Vorrichtung nach Anspruch 6, die weiterhin eine zweite Durchlaßöffnung in dem oberen Teil und einen geschlossenen Flüssigkeitskanal, der die zweite Durchlaßöffnung mit dem Boden des Röhrchens unterhalb des Verengungsteils verbindet, aufweist.

8. Vorrichtung nach Anspruch 1, zur Verwendung in der Isolierung von $CD34^+$-hämatopoetischen Vorläuferzellen, wobei die spezifische Dichte des Zelltrennungsmediums innerhalb ± 0.0005 g/ml der spezifischen Dichte der $CD34^+$-hämatopoetischen Vorläuferzellen liegt.

9. Vorrichtung nach Anspruch 8, wobei das Zelltrennungsmedium eine Osmolalität von 280±10 mOsm/kg $H_2O$ und eine spezifische Dichte von 1.0605 g/ml hat.

10. Vorrichtung nach Anspruch 1, zur Verwendung in der Isolierung von kernhaltigen fötalen Zellen aus einem Zellengemisch, das auch mütterliche Blutzellen enthält, wobei die spezifische Dichte des Zelltrennungsmediums innerhalb von ± 0.0005 g/ml der spezifischen Dichte der fötalen Zellen liegt.

11. Vorrichtung nach Anspruch 10, wobei das Zelltrennungsmedium eine Osmolalität von 280±10 mOsm/kg $H_2O$ und eine spezifische Dichte von 1.0720 g/ml hat.

12. Vorrichtung nach Anspruch 1, zur Verwendung in der Isolierung von Brusttumorzellen aus einem Zellengemisch, wobei das Zelltrennungsmedium eine Osmolalität von 280±10 mOsm/kg $H_2O$ und eine spezifische Dichte ausgewählt aus dem Bereich 1.0490-1.0580 g/ml hat.

13. Verfahren zur Isolierung von ausgewählten Zellen aus einem Zellengemisch, das eine oder mehrere Zelltypen mit Dichten, die unterschiedlich von jener der des ausgewählten Zelltypes sind, enthält, umfassend:

   Einbringen eines Zellgemisches, das den ausgewählten Zelltyp enthält, in eine Zentrifugationsvorrichtung gemäß einem der Ansprüche 1-7, enthaltend ein Zelltrennungsmedium mit einer spezifischen Dichte, die innerhalb ± 0.0005 g/ml der spezifischen Dichte des ausgewählten Zelltypes liegt,
Zentrifugieren in der Vorrichtung bei einer Gravitationskraft, die ausreicht, die Zellen, die eine höhere spezifische Dichte als die spezifische Dichte des Dichtegradientenmaterials in dem Röhrchen haben, zu pelletieren, und
Sammeln einer Zellenfraktion aus dem oberen Teil des Röhrchens der Vorrichtung, die die ausgewählte Zelle enthält.

**14.** Verfahren nach Anspruch 13, wobei das Sammeln durch Dekantieren des Mediums, das im oberen Teil der Vorrichtung vorliegt, aus der Vorrichtung erfolgt.

**15.** Verfahren nach Anspruch 13, das weiterhin die Inkubation des Zellengemisches mit einem zelltypspezifischen Bindemittel umfaßt, das vor der Zentrifugation an Trägerpartikel gebunden wurde, wobei die Partikel eine spezifische Dichte haben, die mindestens 0.001 g/ml größer als die spezifische Dichte des Zelltrennungsmediums ist.

**16.** Verfahren nach Anspruch 15, wobei das zelltypspezifische Bindemittel nicht an den ausgewählten Zelltyp, der isoliert werden soll, bindet.

**17.** Verfahren nach Anspruch 13, zum Anreichern von $CD34^+$-Zellen aus einem Zellengemisch, wobei das Zelltrennungsmedium eine Osmolalität von 280±10 mOsm/kg $H_2O$ und eine spezifische Dichte von 1.0605±0.0002 g/ml hat.

**18.** Verfahren nach Anspruch 13 zur Verwendung bei der Isolierung von kernhaltigen fötalen Zellen aus einem Zellengemisch, das auch mütterliche Blutzellen enthält, wobei das Zelltrennungsmedium eine Osmolalität von 280±10 mOsm/kg $H_2O$ und eine spezifische Dichte von 1.0720±0.0002 g/ml hat.

**19.** Verfahren nach Anspruch 13 zur Verwendung in der Isolation von Brusttumorzellen aus einem Zellengemisch, wobei das Zelltrennungsmedium eine Osmolalität von 280±10 mOsm/kg $H_2O$ und eine spezifische Dichte ausgewählt aus dem Bereich 1.0490-1.0580 g/ml hat.

**20.** Verfahren zur Isolierung von $CD34^+$-Zellen aus einem Zellengemisch, umfassend:

Überschichten des Zellengemisches auf einem Zelltrennungsmedium in einem Zentrifugenröhrchen, wobei das Zelltrennungsmedium eine spezifische Dichte von 1.0605±0.0005 g/ml und eine Osmolarität von 280±10 mOsm/kg $H_2O$ hat,
Zentrifugieren des Zentrifugenröhrchens bei einer Gravitationskraft, die ausreicht, um Zellen, die eine größere spezifische Dichte als die spezifische Dichte des Trennungsmediums haben, zu pelletieren, und
Sammeln der $CD34^+$-Zellen aus dem oberen Teil des Röhrchens.

**21.** Verfahren zur Isolierung von kernhaltigen fötalen Zellen aus einem Zellengemisch, umfassend:

Überschichten des Zellengemisches auf einem Zelltrennungsmedium in einem Zentrifugenröhrchen, wobei das Zelltrennungsmedium eine spezifische Dichte von 1.0720±0.0005 g/ml und eine Osmolarität von 280±10 mOsm/kg $H_2O$ hat,
Zentrifugieren des Zentrifugenröhrchens bei einer Gravitationskraft, die ausreicht, um Zellen, die eine größere spezifische Dichte als die spezifische Dichte des Trennungsmediums haben, zu pelletieren, und
Sammeln der fötalen Zellen aus dem oberen Teil des Röhrchens.

**22.** Verfahren zur Isolierung von Brusttumorzellen aus einem Zellengemisch, umfassend:

Überschichten des Zellengemisches auf einem Zelltrennungsmedium in einem Zentrifugenröhrchen, wobei das Zelltrennungsmedium eine spezifische Dichte von 1.0490-1.0580 g/ml und eine Osmolarität von 280±10 mOsm/kg $H_2O$ hat,
Zentrifugieren des Zentrifugenröhrchens bei einer Gravitationskraft, die ausreicht, um Zellen, die eine größere spezifische Dichte als die spezifische Dichte des Trennungsmediums haben, zu pelletieren, und
Sammeln der Brusttumorzellen aus dem oberen Teil des Röhrchens.

**23.** Verfahren zur Isolierung von natürlichen Killerzellen aus einem Zellengemisch, umfassend:

Überschichten des Zellengemisches auf einem Zelltrennungsmedium in einem Zentrifugenröhrchen, wobei das Zelltrennungsmedium eine spezifische Dichte von 1.0605±0.0005 g/ml und eine Osmolarität von 280±10 mOsm/kg $H_2O$ hat,
Zentrifugieren des Zentrifugenröhrchens bei einer Gravitationskraft, die ausreicht, um Zellen, die eine größere spezifische Dichte als die spezifische Dichte des Trennungsmediums haben, zu pelletieren, und
Sammeln der natürlichen Killerzellen aus dem oberen Teil des Röhrchens.

**Revendications**

**1.** Appareil de séparation de cellules, comprenant :

un tube de centrifugation comportant des parois latérales et un fond fermé;
un élément d'étranglement agencé à l'intérieur du tube, ledit élément d'étranglement étant positionné et construit pour retenir un fluide dans la partie inférieure du tube en dessous de l'élément d'étranglement, lorsque le tube est retoumé, et
un milieu de séparation cellulaire contenu dans la partie inférieure du tube et, avant la centrifugation dudit tube, se prolongeant au-dessus de l'élément d'étranglement susdit jusqu'à un niveau au-dessus d'une ouverture formée par ledit élément d'étranglement, de telle sorte que les cellules qui sont fixées à une interface entre le milieu de séparation cellulaire et un milieu de plus faible densité, après centrifugation, sont déchargées avec le milieu de plus faible densité lorsque le tube est retourné.

**2.** Appareil suivant la revendication 1, dans lequel l'élément d'étranglement précité définit une ou plusieurs surfaces inclinées vers le bas comportant des zones marginales inférieures définissant une ouverture étranglée.

**3.** Appareil suivant la revendication 1, dans lequel l'organe d'étranglement précité est un anneau comportant une ouverture étranglée centrale.

**4.** Appareil suivant la revendication 3, dans lequel ledit anneau est un anneau élastomère agencé pour être ajusté de façon serrée dans le tube à un niveau du tube choisi.

**5.** Appareil suivant la revendication 1, dans lequel l'élément d'étranglement précité définit une pluralité d'ouvertures.

**6.** Appareil suivant la revendication 1, dans lequel le tube susdit comprend une partie supérieure fermée et un orifice dans ladite partie supérieure par lequel une matière fluide peut être introduite dans le tube.

**7.** Appareil suivant la revendication 6, qui comprend de plus un second orifice dans la partie supérieure et un canal de fluide fermé faisant communiquer le second orifice avec le fond du tube en dessous de l'élément d'étranglement précité.

**8.** Appareil suivant la revendication 1, utilisable pour isoler des cellules progénitrices hématopoïétiques $CD34^+$, dans lequel la densité spécifique du milieu de séparation cellulaire précité est dans les limites de ± 0,0005 g/ml de la densité spécifique desdites cellules progénétrices hématopoïétiques $CD34^+$.

**9.** Appareil suivant la revendication 8, dans lequel le milieu de séparation cellulaire a une osmolalité de 280 ± 10 mOsm/kg de $H_2O$ et une densité spécifique de 1,0605 g/ml.

**10.** Appareil suivant la revendication 1, utilisable pour isoler des cellules foetales nucléées d'un mélange de cellules contenant également des cellules sanguines maternelles, dans lequel la densité spécifique du milieu de séparation cellulaire se situe dans les limites de ± 0,0005 g/ml de la densité spécifique desdites cellules foetales.

**11.** Appareil suivant la revendication 10, dans lequel le milieu de séparation cellulaire a une osmolalité de 280 ± 10 mOsm/kg de $H_2O$ et une densité spécifique de 1,0720 g/ml.

**12.** Appareil suivant la revendication 1, utilisable pour isoler des cellules tumorales de sein d'un mélange de cellules, dans lequel le milieu de séparation cellulaire a une osmolalité de 280 ± 10 mOsm/kg de $H_2O$ et une densité spécifique choisie dans la gamme de 1,0490-1,0580 g/ml.

**13.** Procédé d'isolement d'une cellule choisie d'un mélange de cellules contenant un ou plusieurs autres types de cellules avec des densités différentes de celle du type de cellule choisi, comprenant:

l'addition à un appareil de centrifugation suivant l'une quelconque des revendications 1 à 7 et contenant un milieu de séparation cellulaire ayant une densité spécifique qui se situe dans les limites de ± 0,0005 g/ml de la densité spécifique du type de cellule choisi, d'un mélange de cellules contenant le type de cellule choisi,
la centrifugation dudit appareil à une force de gravitation suffisante pour déposer les cellules ayant des densités spécifiques plus élevées que la densité spécifique de la matière en gradient de densité dans le tube précité, et
la collecte de la partie supérieure dudit tube de l'appareil précité d'une fraction cellulaire contenant la cellule choisie.

**14.** Procédé suivant la revendication 13, dans lequel ladite collecte se fait par décantation du dispositif du milieu présent dans la partie supérieure du dispositif.

**15.** Procédé suivant la revendication 13, comprenant de plus l'incubation du mélange de cellules précité avec un agent de liaison spécifique du type de cellule lié à des particules de support avant centrifugation, lesdites particules ayant une densité spécifique qui est d'au moins 0,001 g/ml plus élevée que la densité spécifique du milieu de séparation cellulaire précité.

**16.** Procédé suivant la revendication 15, dans lequel l'agent de liaison spécifique du type de cellule ne se lie pas au type de cellule choisi à isoler.

**17.** Procédé suivant la revendication 13, pour l'enrichissement de cellules $CD34^+$ à partir d'un mélange de cellules, dans lequel le milieu de séparation cellulaire précité a une osmolalité de 280 ± 10 mOsm/kg de $H_2O$ et une densité spécifique de 1,0605 ± 0,0002 g/ml.

**18.** Procédé suivant la revendication 13, utilisable pour isoler des cellules foetales nuclées d'un mélange de cellules contenant également des cellules sanguines maternelles, dans lequel le milieu de séparation cellulaire précité a une osmolalité de 280 ± 10 mOsm/kg de $H_2O$ et une densité spécifique de 1,0720 ± 0,0002 g/ml.

**19.** Procédé suivant la revendication 13, utilisable pour isoler des cellules tumorales de sein d'un mélange de cellules, dans lequel le milieu de séparation cellulaire précité a une osmolalité de 280 ± 10 mOsm/kg de $H_2O$ et une densité spécifique de 1,0490-1,0580 g/ml.

**20.** Procédé d'isolement de cellules $CD34^+$ d'un mélange de cellules, comprenant:

la mise en couche du mélange de cellules sur un milieu de séparation cellulaire dans un tube de centrifugeuse, ledit milieu de séparation cellulaire ayant une densité spécifique de 1,0605 ± 0,0005 g/ml et une osmolarité de 280 ± 10 mOsm/kg de $H_2O$,
la centrifugation du tube de centrifugeuse à une force de gravitation suffisante pour déposer les cellules ayant une densité spécifique plus grande que la densité spécifique du milieu de séparation précité, et
la collecte des cellules $CD34^+$ de la partie supérieure dudit tube.

**21.** Procédé d'isolement de cellules foetales nuclées d'un mélange de cellules, comprenant :

la mise en couche du mélange de cellules sur un milieu de séparation cellulaire dans un tube de centrifugeuse, ledit milieu de séparation cellulaire ayant une densité spécifique de 1,0720 ± 0,0005 g/ml et une osmolarité de 280 ± 10 mOsm/kg de $H_2O$,
la centrifugation du tube de centrifugeuse à une force de gravitation suffisante pour déposer les cellules ayant une densité spécifique plus grande que la densité spécifique dudit milieu de séparation, et
la collecte des cellules foetales de la partie supérieure du tube susdit.

**22.** Procédé d'isolement de cellules tumorales de sein d'un mélange de cellules, comprenant :

la mise en couche du mélange de cellules sur un milieu de séparation cellulaire dans un tube de centrifugeuse, ledit milieu de séparation cellulaire ayant une densité spécifique choisie dans la gamme de 1,0490-1,0580 g/ml et une osmolarité de 280 ± 10 mOsm/kg de $H_2O$,
la centrifugation du tube de centrifugeuse à une force de gravitation suffisante pour déposer les cellules ayant

une densité spécifique plus grande que la densité spécifique dudit milieu de séparation, et
la collecte des cellules tumorales de sein de la partie supérieure du tube susdit.

**23.** Procédé d'isolement de cellules tueuses naturelles d'un mélange de cellules, comprenant :

la mise en couche du mélange de cellules sur un milieu de séparation cellulaire dans un tube de centrifugeuse, ledit milieu de séparation cellulaire ayant une densité spécifique de 1,0605 ± 0,0005 g/ml et une osmolarité de 280 ± 10 mOsm/kg de $H_2O$,
la centrifugation du tube de centrifugeuse à une force de gravitation suffisante pour déposer les cellules ayant une densité spécifique plus grande que la densité spécifique dudit milieu de séparation, et
la collecte des cellules tueuses naturelles de la partie supérieure du tube susdit.

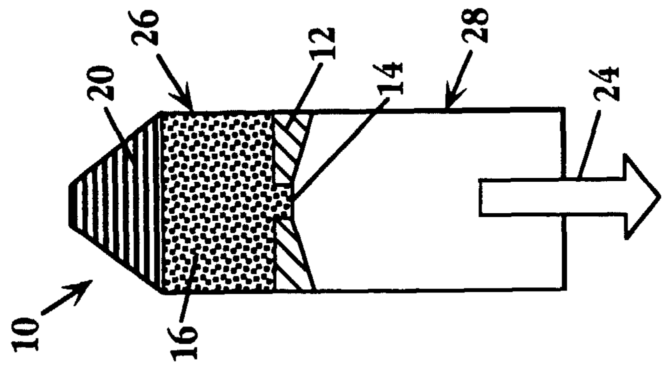


Fig. 1C

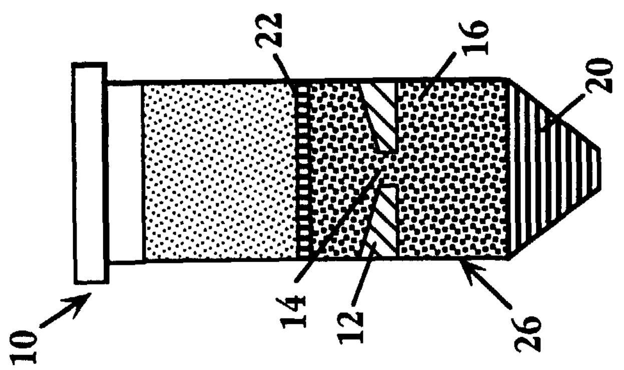


Fig. 1B

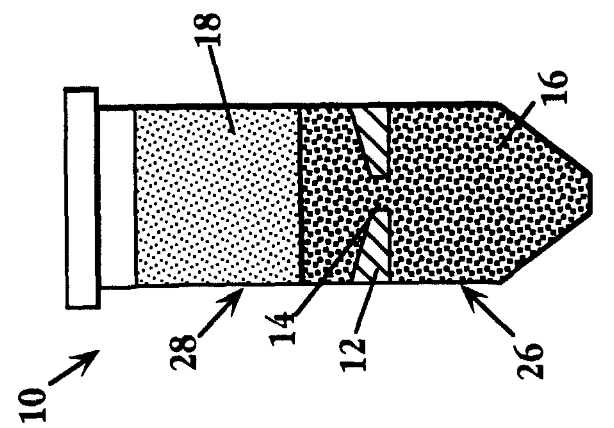


Fig. 1A

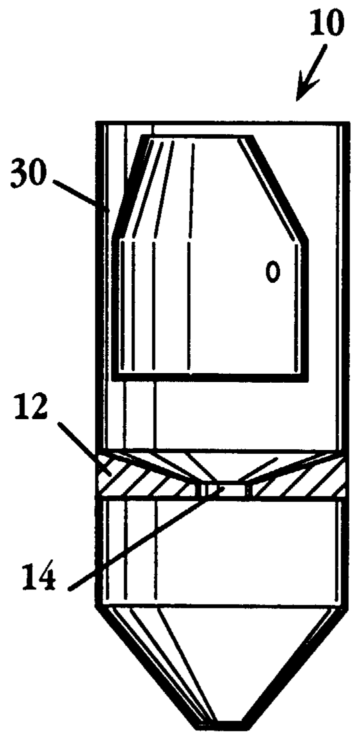


Fig. 2A

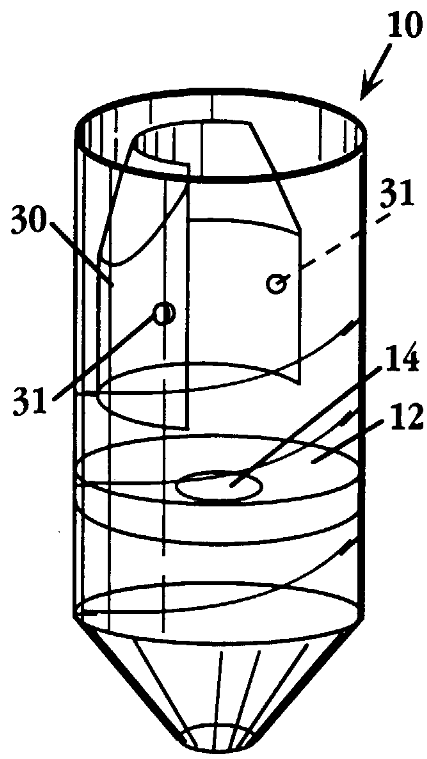


Fig. 2B

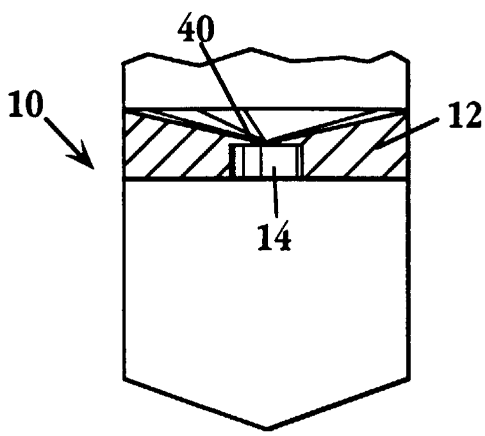


Fig. 3

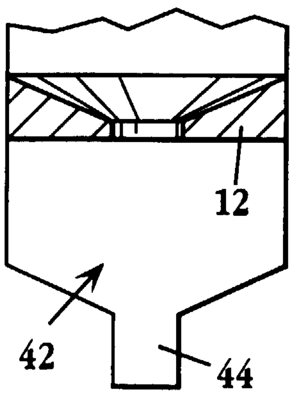


Fig.4A

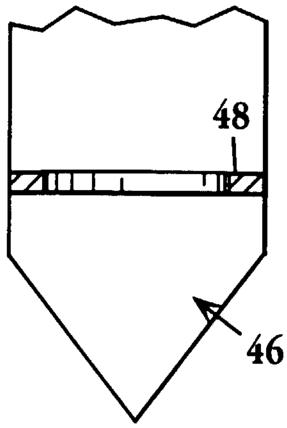


Fig.4B

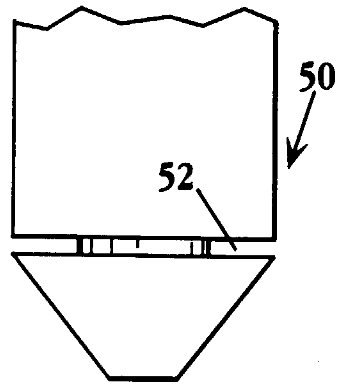


Fig.4C

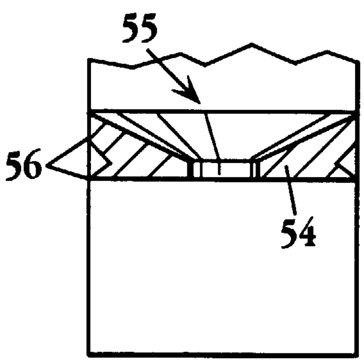


Fig.4D

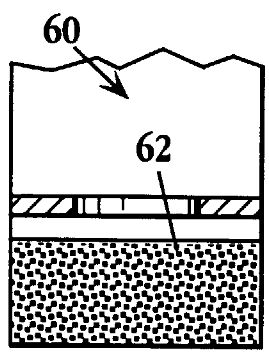


Fig.4E

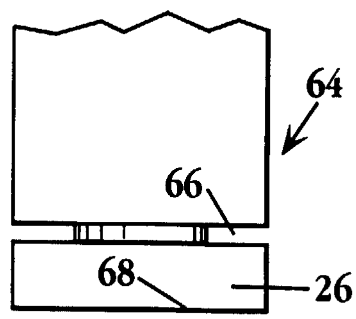


Fig.4F

Fig.5A

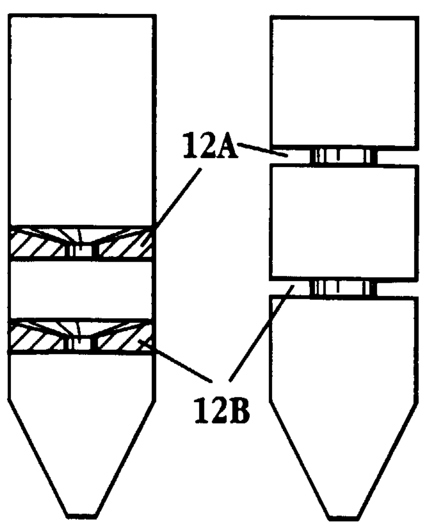


Fig.5B

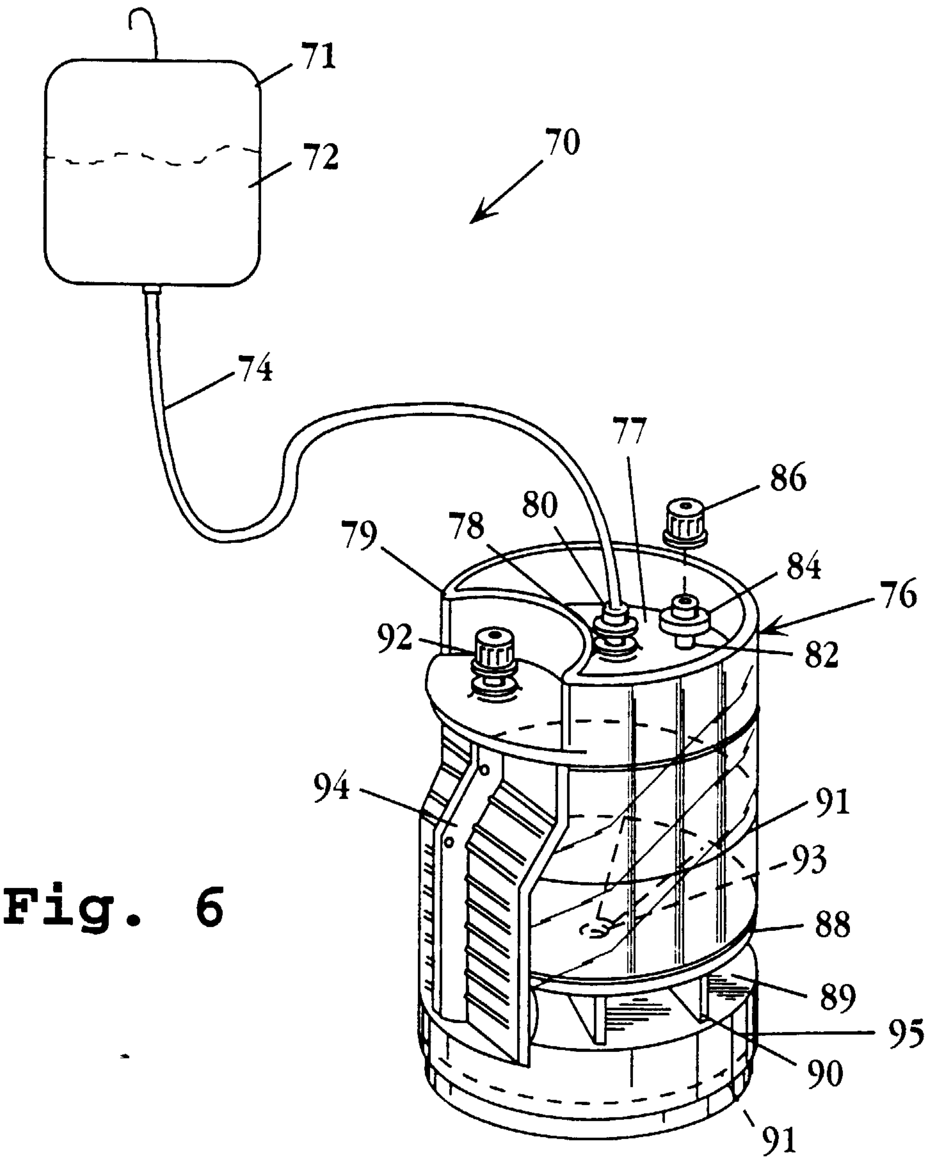


Fig. 6

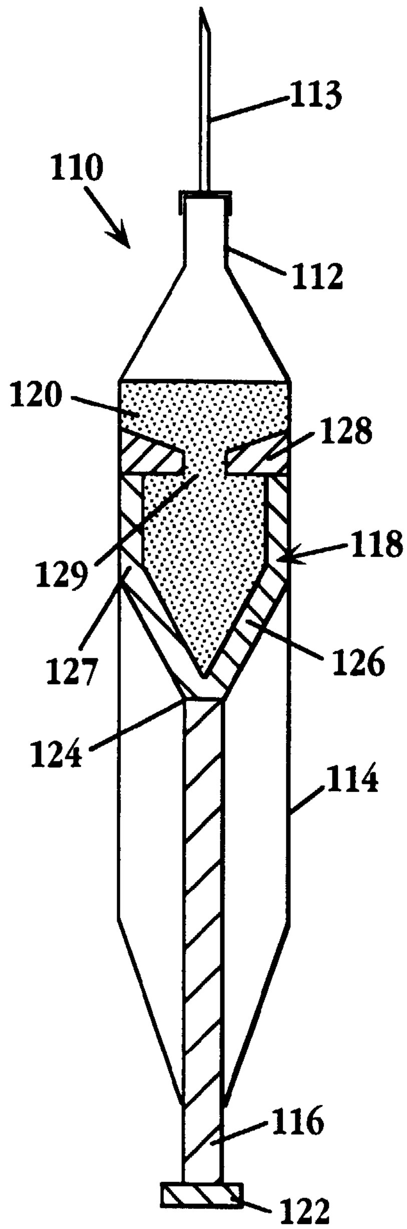


Fig. 7

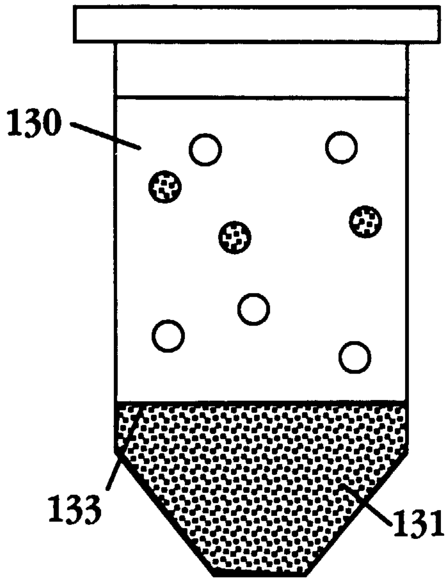


Fig. 8A

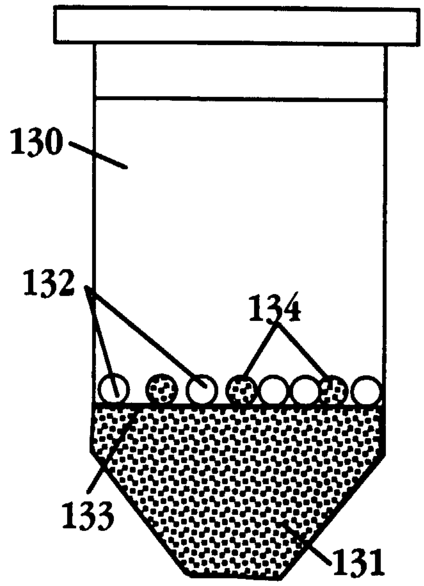


Fig. 8B

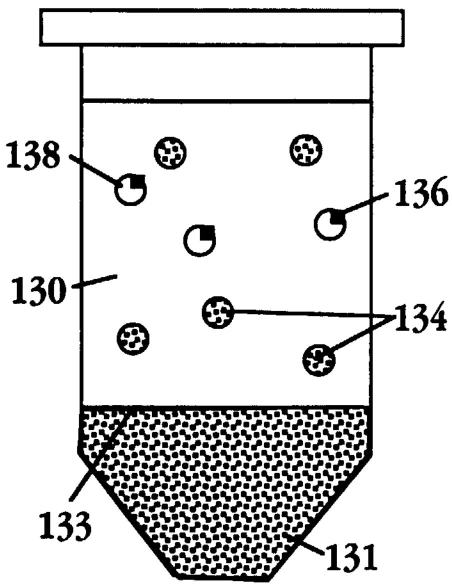


Fig. 8C

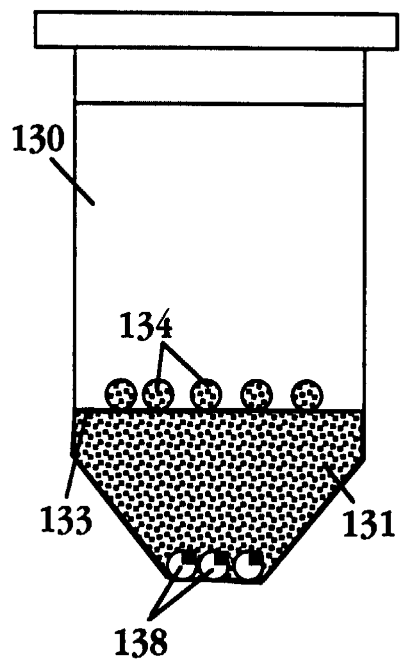


Fig. 8D

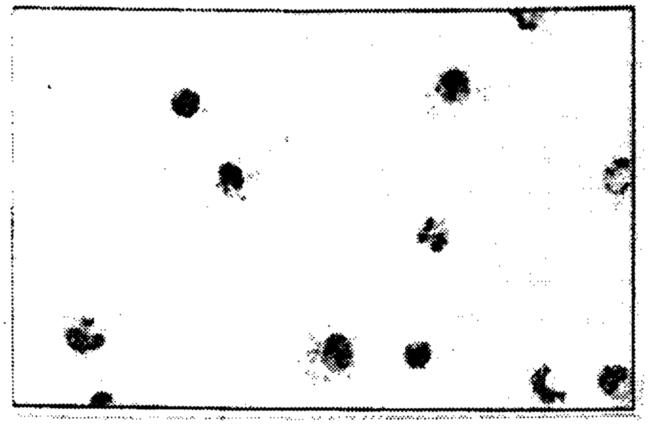

Fig. 9A

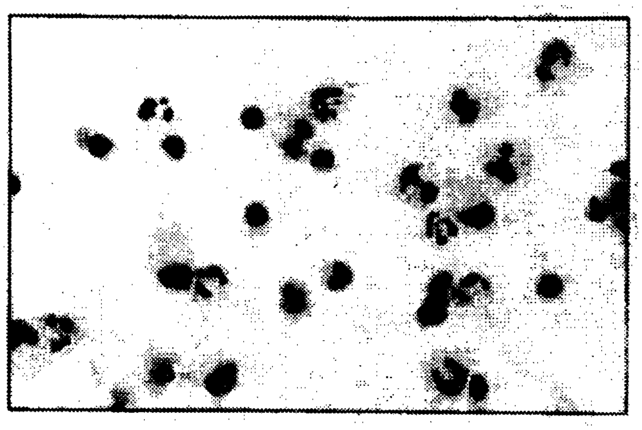

Fig. 9B

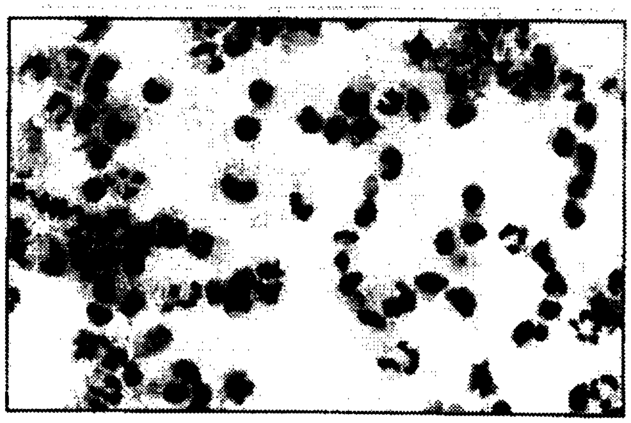

Fig. 9C

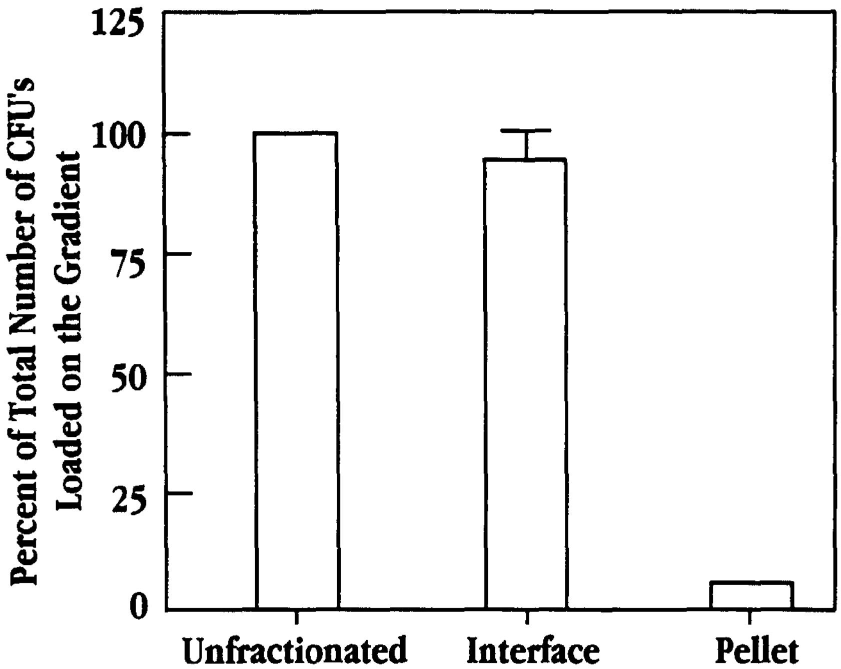
125
100
75
50
25
0
Percent of Total Number of CFU's Loaded on the Gradient
Unfractionated
Interface
Pellet

Fig. 10

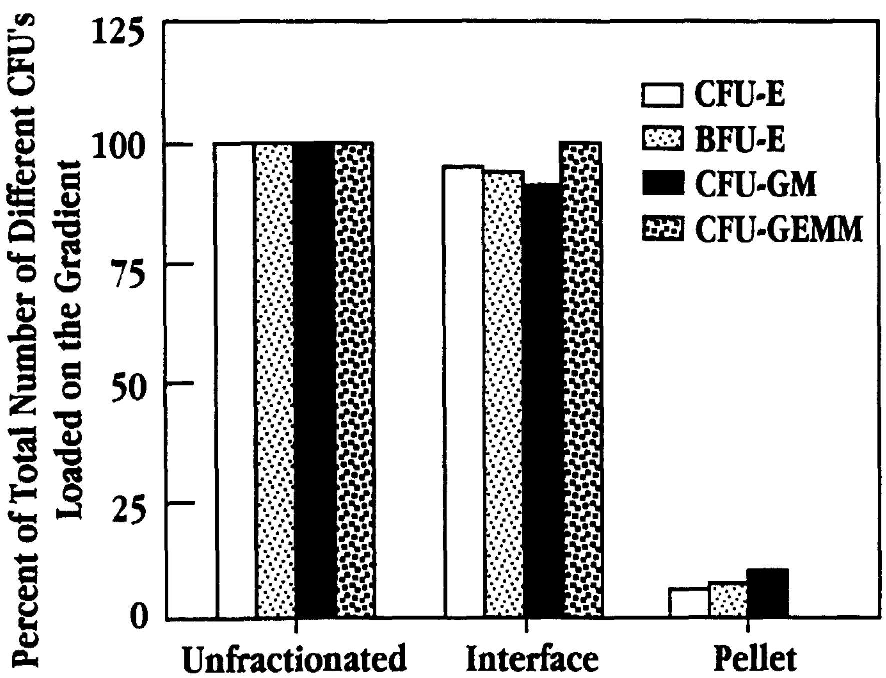
125
100
75
50
25
0
Percent of Total Number of Different CFU's Loaded on the Gradient
CFU-E
BFU-E
CFU-GM
CFU-GEMM
Unfractionated
Interface
Pellet

Fig. 11

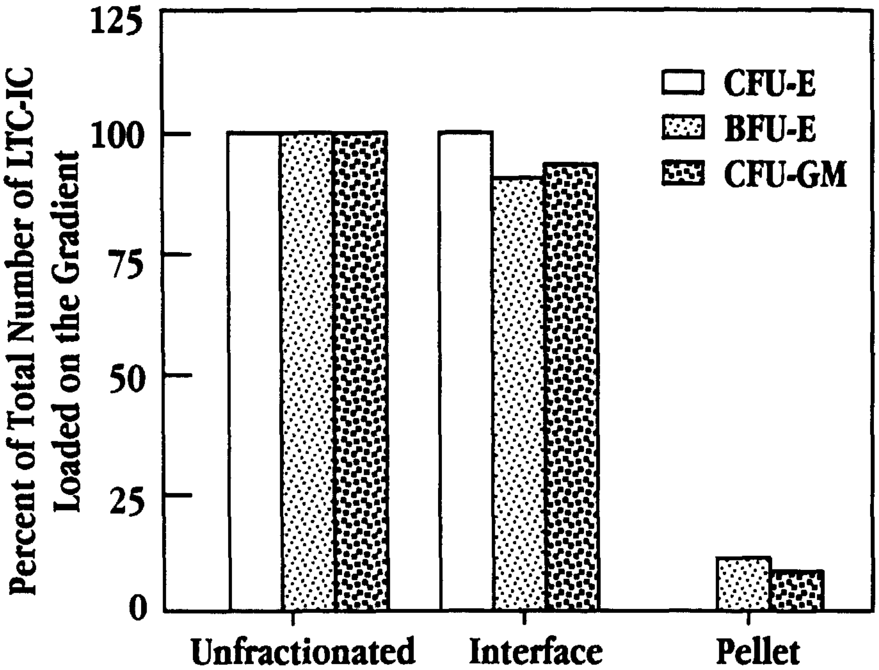
125
100
75
50
25
0
Percent of Total Number of LTC-IC
Loaded on the Gradient
CFU-E
BFU-E
CFU-GM
Unfractionated
Interface
Pellet

Fig. 12

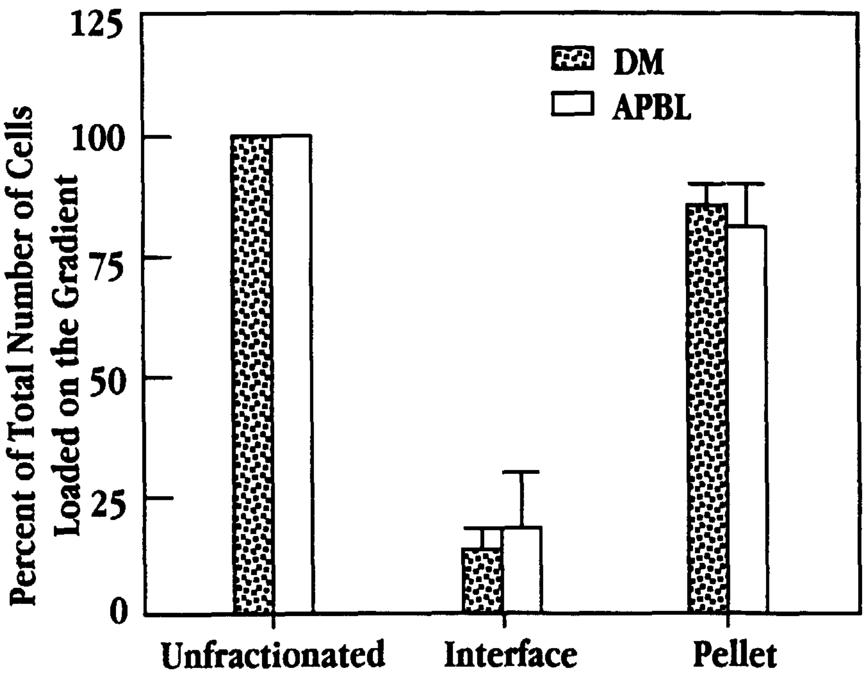
125
100
75
50
25
0
Percent of Total Number of Cells
Loaded on the Gradient
DM
APBL
Unfractionated
Interface
Pellet

Fig. 13

Fig. 14

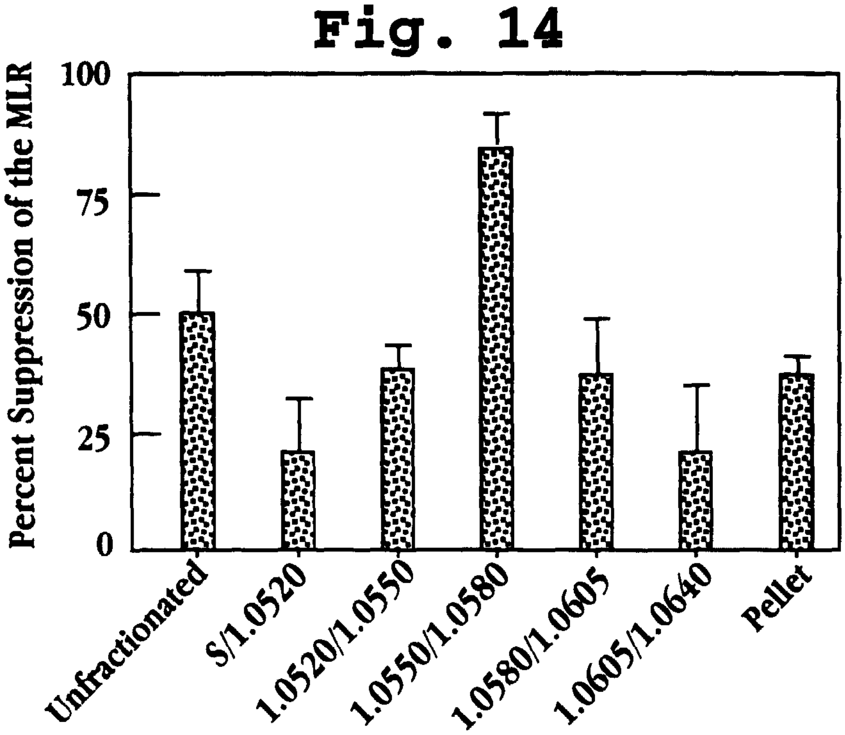
Percent Suppression of the MLR
100
75
50
25
0
Unfractionated
S/1.0520
1.0520/1.0550
1.0550/1.0580
1.0580/1.0605
1.0605/1.0640
Pellet

Fig. 15

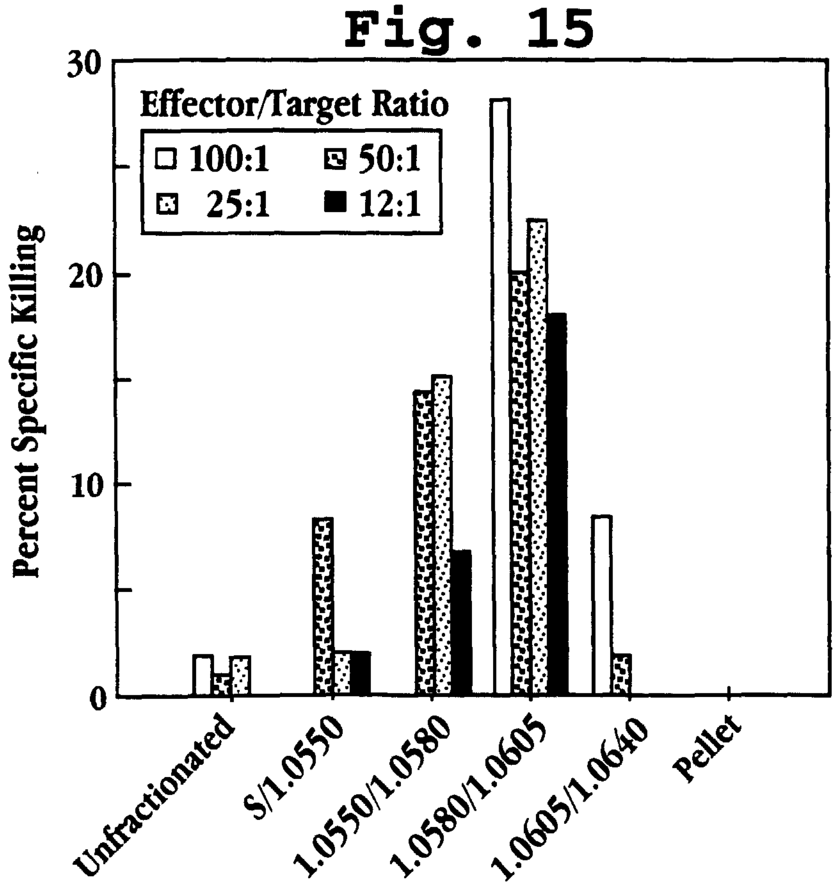
Percent Specific Killing
30
20
10
0
Effector/Target Ratio
100:1
50:1
25:1
12:1
Unfractionated
S/1.0550
1.0550/1.0580
1.0580/1.0605
1.0605/1.0640
Pellet

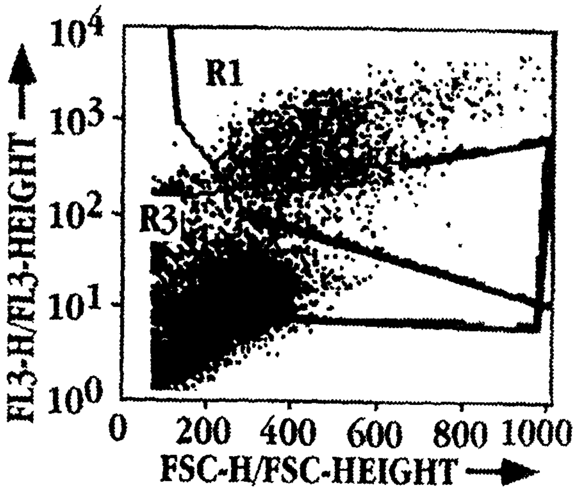


Fig. 16A

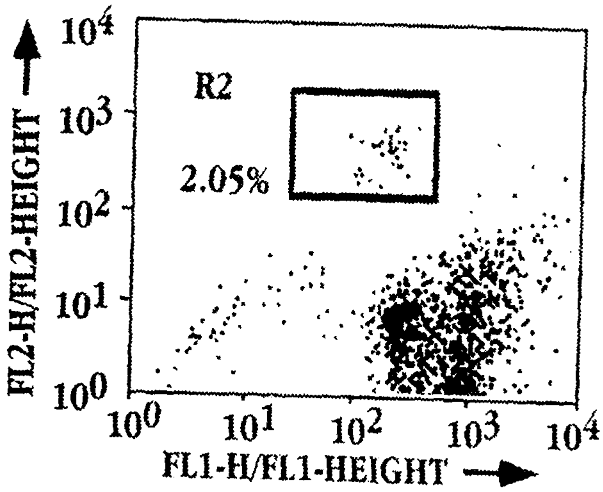


Fig. 16B

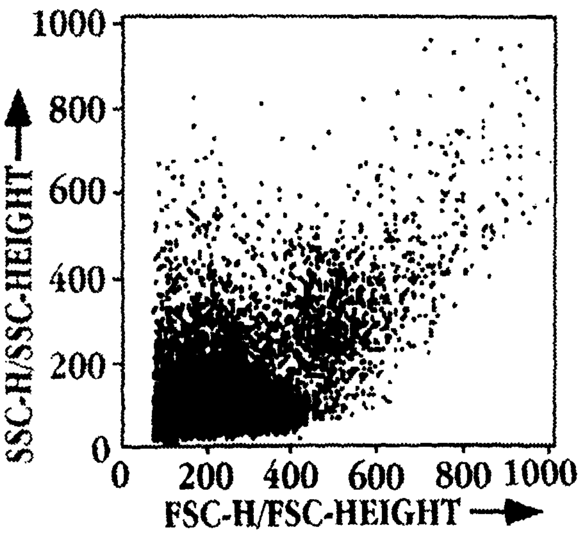


Fig. 16C

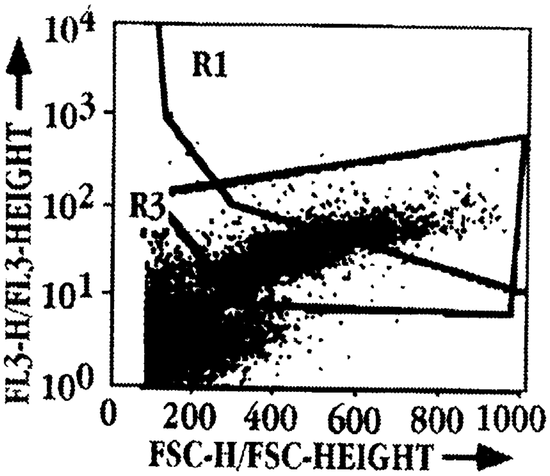


Fig. 16D

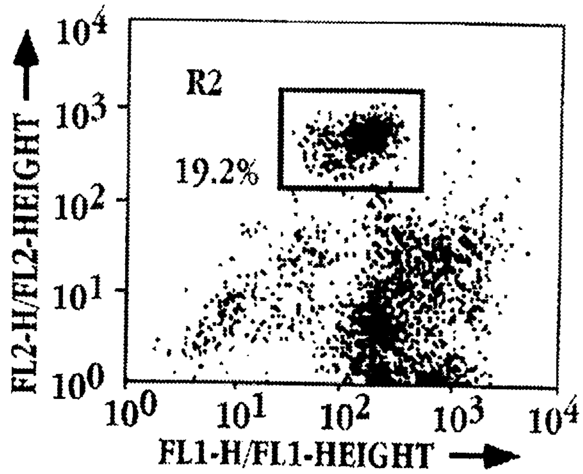


Fig. 16E

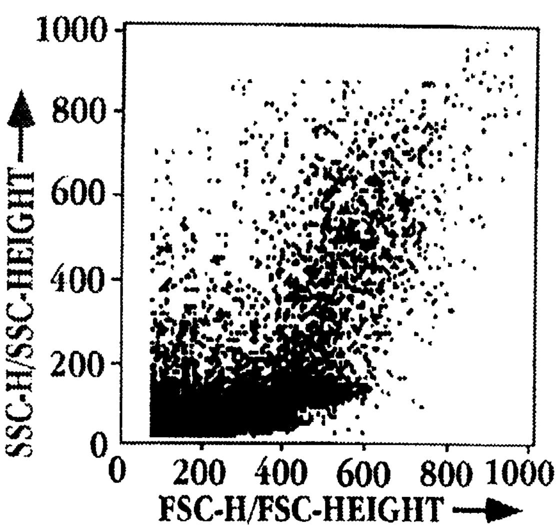


Fig. 16F

Fig. 17A

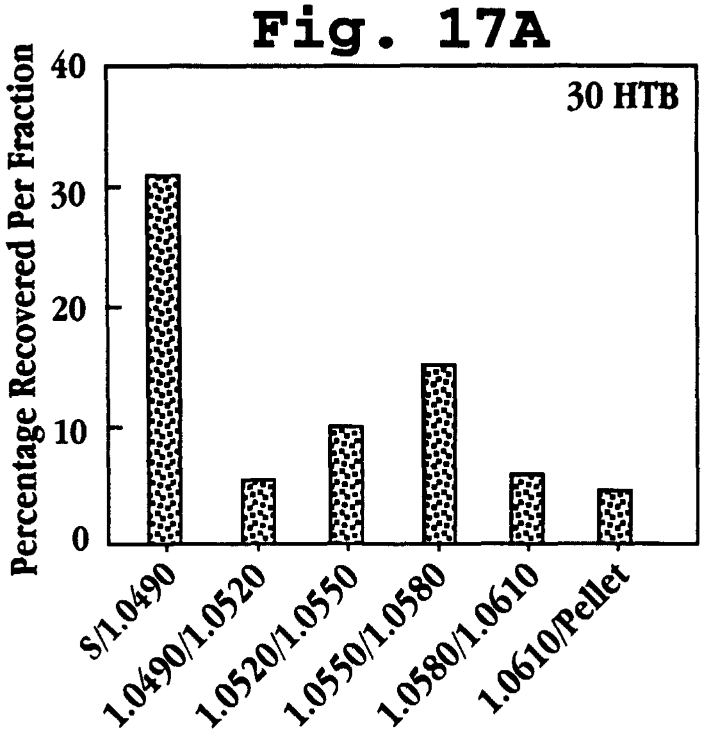


Fig. 17B

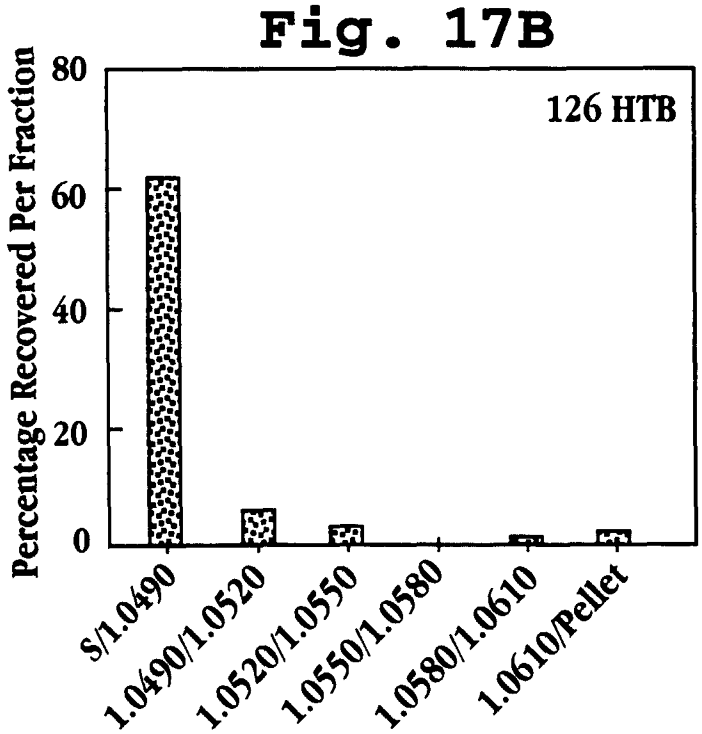

Fig. 17C

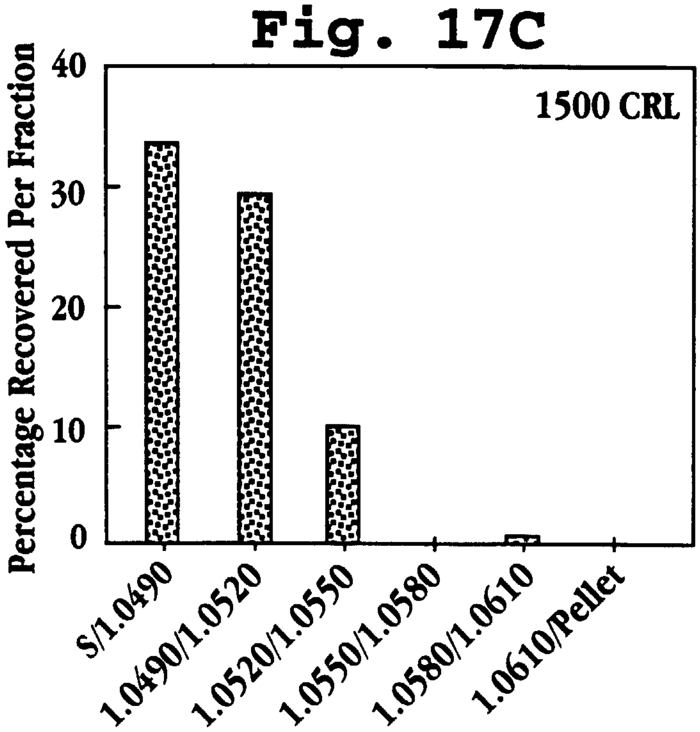


Fig. 17D

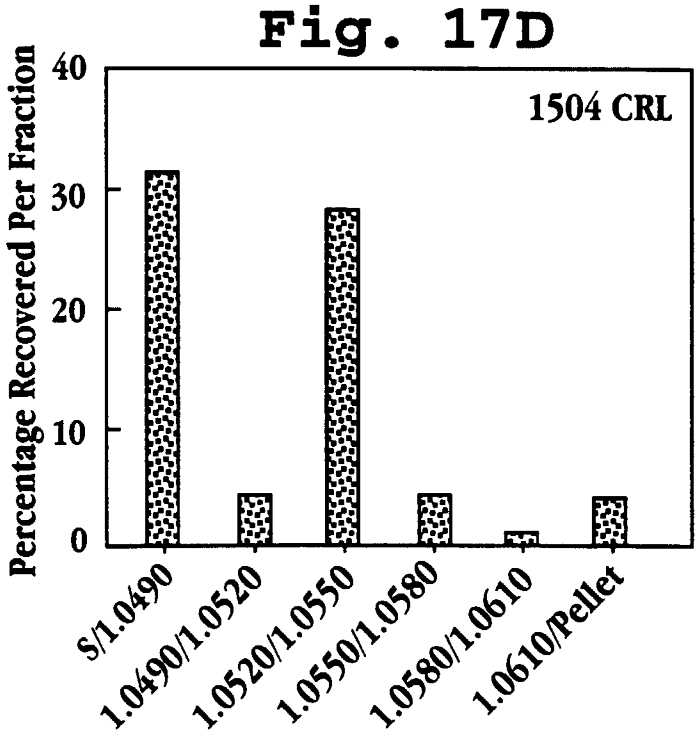

Fig. 18A

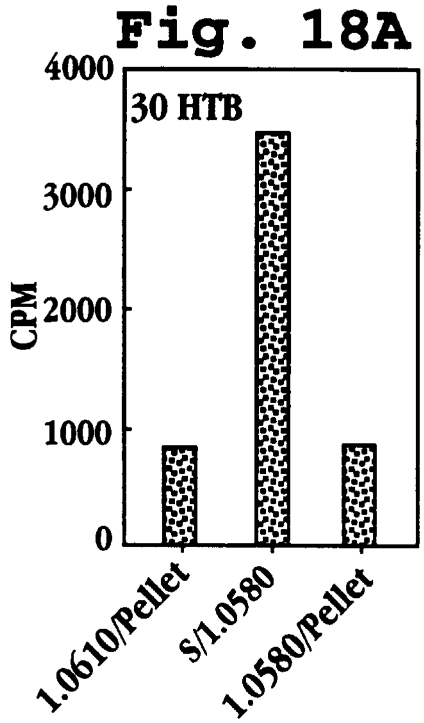


Fig. 18B

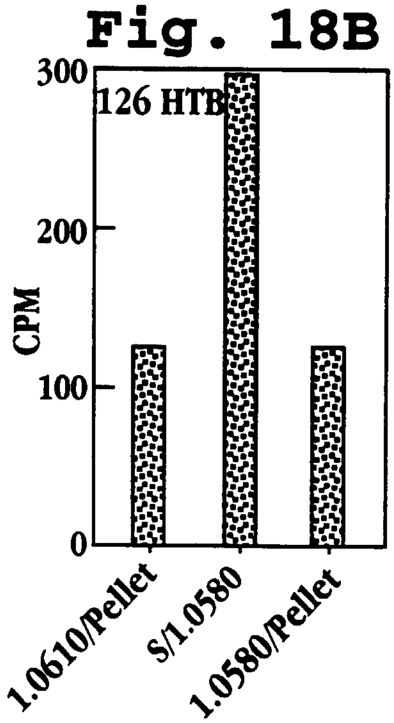


Fig. 18C

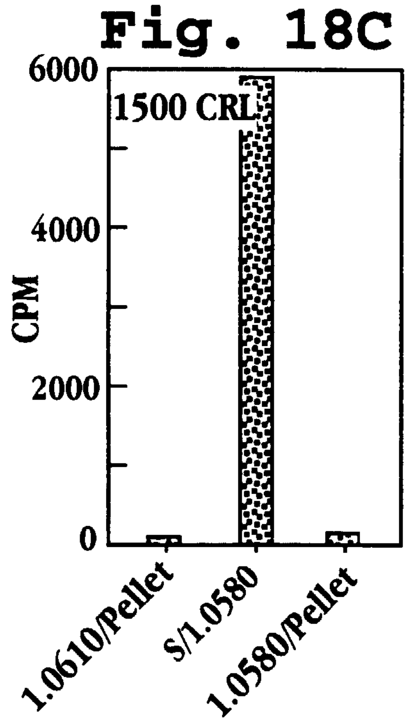


Fig. 18D